(19) Europäisches Patentamt
European Patent Office
Office européen des brevets

(11) **EP 4 570 364 A1**

(12) **EUROPEAN PATENT APPLICATION**

(43) Date of publication:
**18.06.2025 Bulletin 2025/25**

(21) Application number: **23216323.8**

(22) Date of filing: **13.12.2023**

(51) International Patent Classification (IPC):
**B01D 67/00** (2006.01)   **A61M 1/16** (2006.01)
**B01D 69/02** (2006.01)   **B01D 69/08** (2006.01)

(52) Cooperative Patent Classification (CPC):
**B01D 69/02; A61M 1/1621; B01D 67/009;**
**B01D 67/00931; B01D 69/08;** B01D 71/08;
B01D 71/441; B01D 71/68; B01D 2323/34;
B01D 2325/20

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Gambro Lundia AB**
**220 10 Lund (SE)**

(72) Inventors:
• **SWARTJES, Jan J.T.M.**
**915AV Kolham (NL)**
• **VOTTELER, Stefanie**
**72768 Reutlingen (DE)**
• **REMPFER, Martin**
**72810 Gomaringen (DE)**
• **MENDA, Ralf**
**89250 Senden (DE)**
• **STORR, Markus**
**70794 Filderstadt (DE)**

(74) Representative: **Hornung, Veronika Margot**
**c/o Gambro Dialysatoren GmbH**
**Holger-Crafoord-Straße 26**
**72379 Hechingen (DE)**

(54) **HIGHLY SELECTIVE DIALYSIS MEMBRANES AND METHODS FOR PREPARING SAME**

(57)   The present disclosure relates to a membrane with significantly increased selectivity for use in hemodialysis applications, wherein the membrane is coated with chondroitin. The membrane can be obtained by contacting a base membrane, which for example comprises a blend of i) a polysulfone, polyethersulfone or polyarylethersulfone and ii) polyvinylpyrrolidone, to a solution of chondroitin followed by e-beam radiation. The disclosure further relates to a process for producing the selective hemodialysis membrane, and to a method of increasing the selectivity of a membrane. The disclosure further relates to a filtration/diffusion device comprising such membrane.

**EP 4 570 364 A1**

Figure 5

**Description**

**Technical Field**

The disclosure relates to a highly selective membrane for use in hemodialysis applications which is coated with chondroitin. The membrane can be obtained by contacting a base membrane, which for example comprises a blend of i) a polysulfone, polyethersulfone or polyarylethersulfone and ii) polyvinylpyrrolidone, to a solution of chondroitin followed by e-beam radiation. The disclosure further relates to a process for producing the selective hemodialysis membrane, and to a method of increasing the selectivity of a membrane. The disclosure further relates to a filtration/diffusion device comprising such membrane.

**Description of the Related Art**

Patients with end stage renal disease in hemodialysis have an increased mortality risk when compared to the general population. This indicates that the currently available replacement therapies, even though relying on advanced membrane and dialyzer technologies as well as highly effective and sophisticated hemodialysis machines, should be further improved in a way that the clinical outcome for patients and their quality of life during therapy is getting better (Bowry and Chazot, The scientific principles and technological determinants of haemodialysis membranes. Clin Kidney J 14 (Suppl 4): i5-i16 (2021)). One of the still manifold unmet needs that has been accompanying the development of new membranes and dialyzers over decades is the enhanced removal of uremic toxins over a broad range, including higher molecular weight middle molecules, while essentially retaining proteins such as albumin and other higher molecular weight compounds that are deemed to be critical. In other words, synthetic membranes which are state of the art today are still less selective than the glomerular membrane of the kidney. In consequence, uremic toxins having a higher molecular weight are still not cleared appropriately from the patients' blood. In addition, hemodialysis is performed generally thrice weekly for approximately 4 hours, while the healthy kidney operates continuously (Boschetti-de-Fierro et al., Scientific Reports (2015) 5: 18448), which adds to the issues around the accumulation of uremic toxins in the body. Another persistent need in hemodialysis is the hemocompatibility of the materials used in extracorporeal blood treatments, specifically the hemocompatibility of membranes that are in direct blood contact with a significant surface area, making it necessary to use systemic heparin administration in most therapies.

In response to the above challenges, membrane innovation has recently been focussing on enhancing the removal of larger molecular weight uremic toxins and increased membrane permeability. In addition, the industry also focused on the improvement of hemocompatibility and antithrombogenicity of the membranes. The progressive shift towards higher molecular weight toxins and increased membrane permeability also necessitates the provision of membranes with an increasingly improved selectivity. In other words, it remains a major target in the industry to close the gap between synthetic membranes and the natural kidney by combining high permeability and high selectivity.

Typical hallmarks of selectivity of a membrane specifically for use in hemodialysis are the sieving coefficients for albumin (~66.4kDa) on the one hand and the sieving coefficients for large middle molecules (>25-58kDa) on the other hand (Rosner et al., Classification of Uremic Toxins and Their Role in Kidney Failure: CJASN 16, 1-8 (2021)). For albumin, the sieving coefficients should be very low, whereas those for large middle molecules should be high. The challenges resides in providing membranes that are able to combine both requirements, which is nothing else than high selectivity. Large middle molecules include, for example, YKL-40 (~40kDa), AGEs, lambda-FLC, CX3CL1, CXCL12, or IL-2. However, selectivity does expressly encompass the highly efficient removal of also small middle molecules (0.5-15kDa) such as $\beta$2-microglubulin, and medium middle molecules (>15-25kDa), such as TNF, IL-10, IL-6, kappa-FLC, myoglobin, FGF-2, complement factor D and others. The sieving coefficients for said molecules are reflected, for example, in the sieving curve represented by the MWCO and MWRO values of a membrane. These parameters are a means to provide information on the onset of retention of molecules, i.e., where the sieving coefficient is 0.9, and the cut-off of a membrane, i.e., where the sieving coefficient is 0.1.

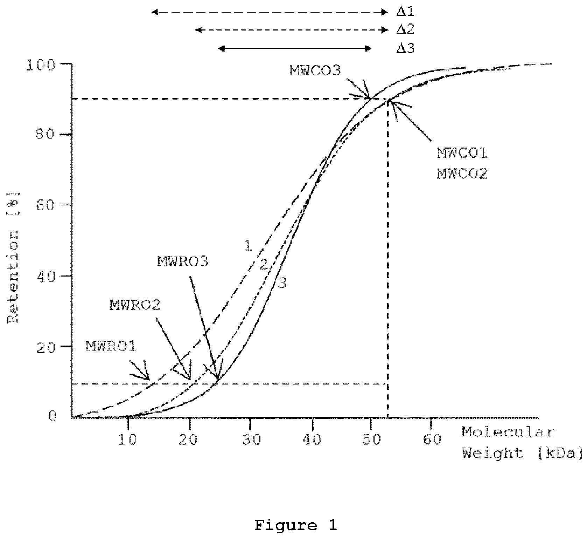

Together, MWRO and MWCO define the steepness of the sieving curve and thus provide for an excellent measure of the selectivity of a membrane. For example, shifting the retention onset to higher molecular weights while keeping the cut-off stable or reducing it, results in increased selectivity, which is apparent from an increasing steepness of the sieving curve which is determined by the molecular weight retention onset (MWRO) and the molecular weight cut-off (MWCO) of a membrane (**FIG. 1**). In other words, an increase in selectivity is defined by a decrease of the delta $\Delta$ between MWRO and MWCO of a membrane, or by an increase of the absolute value of the slope at the turning point of the sieving curve. Keeping the MWRO of a membrane stable while reducing the MWCO thereof could be another way to improve the selectivity of a membrane. However, it is a challenge to achieve that desired shift of either MWRO or MWCO while keeping the respective other parameter stable.

Hemodialyzers comprising such selective membranes should have a low albumin loss over a typical dialysis treatment of about 4 hours (or simulated use correlated with *in vivo* use), i.e., the albumin loss should remain below about 7 g/4h, and preferably should remain below about 4g/4h. At the same time, the hemodialyzers should provide for a good clearance for large middle molecules, including, for example, YKL-40, i.e., they should have a YKL-40 clearance of at least 20 mL/min and higher. They should, in addition, efficiently remove $\beta$-2 microglobulin ($\beta$2m) with a clearance of more than 80 mL/min.

$\beta$-2 microglobulin is a well-known marker molecule in hemodialysis. It is a component of MHC class I molecules with a molecular weight of 11 kDa that in patients on long-term hemodialysis can aggregate into amyloid fibers that deposit in joint spaces, a disease, known as dialysis-related amyloidosis. YKL-40 (or chitinase-3-like protein) has recently emerged as a relevant and functional marker protein in hemodialysis (Lorenz et al., Kidney International (2018) 93: 221-230), where it has been linked to chronic inflammatory response in chronic kidney disease and HD patients. Its presence in the serum of the patients makes it accessible and traceable.

Recently, a significant step towards membranes and hemodialyzers with such improved performance was made by the introduction of so-called medium cut-off membranes and dialyzers, which are characterized by membranes having a high molecular weight retention onset (MWRO), thereby allowing also larger molecular weight toxins to pass the membrane to a significant extent, combined with a low molecular weight cut off (MWCO), meaning that proteins and other compounds that have a molecular weight of albumin and higher are essentially retained (WO 2015/118046 A1 and WO 2015/118046 A1). In other words, the design of said medium cut-off membranes allows for a significantly improved selectivity, which is a distinguishing feature over other prior art membranes such as the so-called "high cut-off" membranes, or membranes that have been referred to, in the past, as "protein-leaking" (PL) membranes. While high cut-off membranes are also able to efficiently remove higher molecular weight toxins, their increased albumin loss recommends their use only in acute situations that are limited in time and strictly controlled as regards albumin loss and replacement thereof. Protein-leaking membranes, on the other hand, generally have a cut-off that provides for a limited loss of albumin and other higher molecular weight compounds beyond albumin, but the retention onset is generally low, resulting in a flat sieving curve that signifies a low selectivity and undesirable weakness in the clearance of smaller and of large uremic toxins. In contrast, medium cut-off membranes when combined with a careful physical design of the hollow fibers, membrane bundle and overall dialyzer, lead to a performance which provides for an unprecedented clearance of relevant higher molecular weight toxins in hemodialysis, while albumin loss can be kept within narrow limits (Kirsch et al., Nephrology Dialysis Transplantation (2017) 32: 165). A commercially available example for such a medium cut-off membrane and dialyzer is the MCO Theranova dialyzer (Baxter).

The significant contribution of medium cut-off membranes to the final superior dialyzer performance is achieved by their unique membrane structure which is generated by a careful control of all relevant parameters of the manufacturing process as described in WO 2015/118045 A1 and WO 2015/118046 A1. Such highly controlled manufacturing process is not easily reproducible throughout the industry, and further improving the selectivity of medium cut-off membranes by further improving the process is difficult and/or expensive. However, as mentioned above, it would be desirable to further improve dialysis membranes towards even higher selectivity by easily accessible methods that allow the production of highly selective hemodialysis membranes without excessive production costs that add to the price of the final product.

Other membranes, such as standard hemodialysis membranes and dialyzers, including, for example, high cut-off as well as high-flux membranes that are today widely used in hemodialysis (HD) could especially benefit from methods to improve their selectivity in a simple, cost-effective, and safe way to provide access to improved dialyzers to more patients that may otherwise not be able to benefit from potentially more expensive high-end dialyzers as discussed above or potentially even more expensive and complex therapies such as HDF. Of course, any such methods for improving the selectivity of available membranes and dialyzers must guarantee the biocompatibility of the membrane and the dialyzer and its general safety for the patient (Bowry and Chazot (2021): The scientific principles and technological determinants of haemodialysis membranes. Clin Kidney J 14 (Suppl 4): i5-i16).

Technologies for the modification of membranes, including hemodialysis membranes, by incorporating or immobilizing biologically active or inactive compounds in/on the membrane have generally been described in the prior art. This includes, for example, modifications for improving their biocompatibility, antithrombogenicity, or performance. They further encompass adding compounds to the membrane matrix for the unspecific capturing of, for example, hydrophobic or hydrophilic uremic toxins. Modifications also include methods for specifically functionalizing membrane surfaces for the binding of, for example, antibodies that can specifically target molecules that are causative for various diseases and which are thus removed from the blood of the patient.

Chondroitin is a compound which has already been used in the past for modifying structures or materials of medical devices as it can safely be used as a component for medical applications (Miraglia et al. (2016) Food and Chemical Toxicology 93: 89-101). Chondroitin, or chondroitin sulfate (herein generally referred to as "CS"), is an anionic linear polysaccharide, which is synthesized as part of proteoglycan (PG) molecules in vertebrates and invertebrates. CS, with different disaccharides and overall carbohydrate backbones, may be biosynthesized possessing various numbers and positions of sulfate groups. Consequently, CS is a relatively heterogeneous molecule having different molecular weights and being formed of alternate and variously sulfated disaccharides of GlcA and GalNAc linked by $\beta(1{\to}3)$ bonds. Moreover, the different disaccharides can be linked to each other by $\beta(1{\to}4)$ bonds. CS-A and CS-C are the predominant CS variants and are constituted by disaccharides sulfated in position C4 or C6 of the GalNAc residue, respectively. Minor percentages of the monosulfated disaccharide on C2 of GlcA exist as well. CS-A or CS products containing predominantly CS-A (as used herein, this expression means that more than 50%, 60%, 70%, 80%, 90% or 95% of the CS are of the CS-A type) may be preferable in connection with the present invention.

Besides the main presence of these two kinds of disaccharide units monosulfated in position C4 or C6 of GalNAc, disaccharides with a different number and position of sulfate groups can be present, in various percentages, within the carbohydrate chains (Volpi, molecules (2019), 24: 1447; Awofiranye et al., Fermentation (2022), 8: 323). CS is mostly produced from the cartilage of animals such as cows, pigs, chicken, or marine organisms and therefore subject to certain variations depending on the species on the process used for extraction. However, it can also be produced synthetically or by fermentation which has a benefit in terms of purity and homogeneity. An example of fermentation-derived CS is CS from Mythocondro.

In Ning et al., Progress in Polymer Science (2018), 81: 144-162, polypyrrole (PPy) nanostructures for use as regenerative biomaterials in tissue regeneration were doped with, for example, chondroitin sulfate (CS).

WO 2017/030502 A1 describes biomimetic membranes for use in nanofiltration. The membranes have deposited thereon a first mixture comprising a first polyelectrolyte and a transmembrane protein such as aquaporin, and a second mixture comprising a second polyelectrolyte and a cross-linking agent, wherein the second polyelectrolyte has a charge opposite to the charge on the first polyelectrolyte. Chondroitin sulfate is mentioned as an anionic polyelectrolyte which can be used for producing such biomimetic membranes.

Ren et al., Chemical Engineering Journal (2019), 370: 1027-1038, describe aligned porous poly (l-lactic acid) (PLLA) electrospun fibrous membrane with a biomimetic surface for the rapid regeneration of cartilage tissue. The PLLA electrospun fibers aligned in a single direction and generated ellipse-shaped nanopores in situ onto the surface of the fibers. Subsequently, chondroitin sulfate (CS) was coated on the surface of the fibers using polydopamine (PDA) as an adhesive polymeric bridge.

WO 2023117812 A1 discloses hemodialysis membranes prepared from a blend comprising polysulfone, polyethersulfone or polyarylethersulfone, and polyvinylpyrrolidone, wherein the membrane is coated with polydopamine and chondroitin, and optionally with heparin as a third component. WO 2023117812 A1 for the first time discloses that a coating with polydopamine and chondroitin, optionally in combination with heparin, can improve the selectivity of a membrane. It is also mentioned that e-beam irradiation is a way to sterilize the membrane as the method does not negatively impact the desired effects achieved with the proposed coating. However, polydopamine and chondroitin as well as, where present, heparin, may be eluted from the membrane surface, thereby entering the blood stream of the patient. Even though only small amounts of polydopamine and chondroitin are "leaking" from the membrane, and even though both are non-toxic, it would be preferable to avoid such membrane behavior to avoid any substances entering the patient's blood stream.

Finally, Schulze et al., Macromolecular Rapid Communications (2010) 31:467-472, showed that polyethersulfone and polyacrylonitrile membranes were surface modified in a one-step procedure, leading to the stable immobilization of certain surface modifying substances. For this purpose, the membranes were soaked with aqueous solutions of different low-molecular weight molecules bearing diverse hydrophilic functionalities and subject to electron beam treatment. No catalysts, photoinitiators, organic solvents or other toxic reagents were used, and no additional synthetic or purification steps were required. The concentrations of various molecules varied in the range of from 0.1-2.0% and irradiation doses in the range of 30-300kGy were applied. Irradiation was performed in an $N_2$-atmosphere with $O_2$ quantities. Chondroitin, however, is not mentioned in Schulze et al. (2010). Also, the reference is silent on the effects of e-beam radiation in combination with any of the substances used on the selectivity of membranes.

It was now found that the selectivity of a base membrane as used, for example, in hemodialysis applications, including membranes based on polysulfone, polyethersulfone or polyarylethersulfone, and polyvinylpyrrolidone, can surprisingly be enhanced with a simple coating with chondroitin (CS), if the membrane after coating with a solution having a CS concentration of from 0.1 wt.% to 10 wt.-% (or 1 mg/mL - 100 mg/mL) is e-beam treated at doses of from 12.5 kGy to 115 kGy. Coating solutions with 3 wt.% to 8 wt.% are especially suitable for preparing membranes according to the invention. No elution of chondroitin is observed after e-beam treatment, while the selectivity of a given membrane is significantly increased by the combination of chondroitin alone and e-beam irradiation.

Other characteristics relevant for hemodialysis applications such as, for example, biocompatibility, or technical parameters such as, for example, Lp, are not negatively affected, making the combination of chondroitin coating in combination with e-beam irradiation a surprisingly effective and simple way to increase the pre-existing selectivity of a given membrane.

## Summary

It is an object of the present invention to provide hollow fiber and flat sheet membranes for use in separation and/or blood treatment applications such as, for example, hemodialysis, hemofiltration or hemodiafiltration. Said membranes can be obtained by contacting a base membranes with a solution of chondroitin followed by submission to e-beam irradiation. As used herein, the expression "membrane" or "membranes" encompasses both hollow fiber membranes and flat sheet membranes. In the context of the present invention, hollow fiber membranes are preferred as they constitute the state-of-the-art membrane configuration, e.g., for use in hemodialysis devices.

According to one aspect of the present invention, a membrane for use in extracorporeal blood treatment applications comprises a base membrane, such as, for example, a membrane comprising a blend of a polysulfone (PS), polyethersulfone (PES), or polyarylethersulfone (PAES), and polyvinylpyrrolidone (PVP), and a coating which comprises chondroitin. The membrane is prepared by treating the base membrane with an aqueous solution comprising from 0.1 wt.-% to 10.0 wt.-% of chondroitin, followed by submitting the coated membrane to e-beam radiation at a dose of from 12.5 kGy to 115 kGy. The membrane is characterized in that the coated membrane has a higher selectivity in comparison with the base membrane. A higher selectivity can be expressed in a smaller difference between MWRO and MWCO of a coated membrane in comparison with an uncoated membrane (see also **Fig. 1**)

According to one aspect of the invention, the base membrane before coating comprises from 0.5 wt.-% to 5.0 wt.-% PVP and from 95.0 wt.-% to 99.5 wt.-% PES, PS, or PAES.

According to another aspect of the invention, the base membrane is treated with an aqueous solution comprising from 0.1 wt.-% to 8.0 wt.-% chondroitin sulfate.

According to yet another aspect of the invention, the base membrane is coated with chondroitin sulfate in an amount of from 0.30 $\mu$g/cm$^2$ to 2.00 $\mu$g/cm$^2$ of the membrane area.

According to another aspect of the invention, the base membrane is coated with chondroitin sulfate which consists of or predominantly comprises chondroitin sulfate of the CS-A type (chondroitin-4-sulfate), wherein "predominantly comprises" refers to CS mixtures (e.g., mixtures with CS-C) that comprise more than 50% CS-A, preferably more than 60% CS-A, more than 70% CS-A, more than 80% CS-A, more than 90% CS-A, or more than 95% CA-A.

According to another aspect of the invention, the base membrane is coated with chondroitin sulfate which consists of or predominantly comprises chondroitin sulfate of the CS-C type (chondroitin-6-sulfate), wherein "predominantly comprises" refers to CS mixtures (e.g., mixtures with CS-A) that comprise more than 50% CS-C, preferably more than 60% CS-C, more than 70% CS-C, more than 80% CS-C, more than 90% CS-C, or more than 95% CA-C.

According to another aspect of the invention, the base membrane is coated with chondroitin sulfate which has an average molecular weight (MW) of from 10 kDa to 60 kDa, preferably from 10 kDa to 50 kDa, and especially preferably from 10 kDa to 30 kDa or from 20 kDa to 50 kDa. According to another aspect of the invention, the chondroitin sulfate used is chondroitin sulfate A sodium salt with an average MW of from 10 kDa to 50 kDa or from 20 kDa to 30 kDa, or chondroitin sulfate C sodium salt with an average molecular weight of from 1 to 5 kDa.

According to another aspect of the invention, the membrane after being contacted with an aqueous solution comprising chondroitin sulfate is submitted to e-beam radiation at a dose of from 12.5 kGy to 110 kGy, preferably at a dose of from 20 kGy to 100 kGy, such as from 25 kGy to 100 kGy or from 50 to 90 kGy, such as, for example, 75 kGy.

According to a further aspect of the invention, e-beam radiation can be done either in the presence of the coating solution or after incubation of the base membrane with a chondroitin solution according to the invention, followed by removal of the solution and optionally rinsing the membrane with an aqueous solution. Preferably, e-beam radiation is done in the presence of the coating solution (referred to herein as the "filled" state, as the membrane will be provided together with the coating solution inside of a container that is, accordingly, "filled" with the coating solution surrounding the membrane). The coating solution will generally be removed from the membrane/filter device after e-beam radiation. The membrane and/or filter device can be rinsed with water one or several times and then be submitted to final sterilization, e.g., by renewed e-beam irradiation or by gamma irradiation according to known practices.

According to another embodiment of the invention, membranes that are preferably used as base membranes can have different physical structures, including varying degrees of asymmetric configuration, ranging from minimum asymmetry in sponge-like structures to maximum asymmetry in finger-like structures, such as described, for example, in Ronco and Clark, Nature Reviews. Nephrology 14 (6) (2018): 394-410. In some applications the lumen or inner side of the hollow fibers membranes is coated as it generally provides for the selective layer of the hollow fiber membranes in hemodialysis applications, wherein the lumen is in contact with blood, or, optionally, with blood plasma. The "lumen" surface of the membrane has pores which may extend from said lumen side of the hollow fiber membrane towards the other side of the membrane. However, devices wherein the outer surface of hollow fiber membranes is in contact with blood whereas the lumen is transfused with a solution (e.g., a dialysis solution) or gas are also known. In devices where the outer side of a hollow fiber membrane is in contact with blood and provides for the selective layer of the membrane, a modification or coating according to the invention would preferably be applied to the outer side of the hollow fiber membranes. If so desired, the complete membrane can be treated with a CS solution and will then be coated on the outer surface as well as on the lumen side of the hollow fiber membrane, including the accessible surfaces of the pores of the membrane.

Accordingly, it is one aspect of the present invention that the base membrane, which is a hollow fiber membrane, is coated with chondroitin on the lumen side of the membrane including any adjacent/accessible pores. According to another aspect, the base membrane, which is a hollow fiber membrane, is coated with chondroitin on the outer surface of the hollow fiber membrane including adjacent/accessible pores. According to yet another aspect of the invention, the complete surface of a hollow fiber membrane is coated with chondroitin, including the surfaces of accessible pores.

According to one aspect, the base membrane is a medium cut-off membrane and has a MWRO of from 8.5 to 14.0 kDa and a MWCO of from 50.0 to 130.0 kDa as measured by dextran sieving before blood contact, preferably a MWRO of from 8.5 to 12.5 kDa and a MWCO of from 55.0 to 110.0 kDa as measured by dextran sieving before blood contact. According to yet another aspect, the base membranes have a MWRO of from 9.0 to 12.5 kDa and a MWCO of from 55.0 to 90.0 kDa as measured by dextran sieving before blood contact. Such membranes are generally referred to as medium cut-off membranes, sometimes also as high retention onset or HRO membranes. They are described in detail in WO 2015/118045 A1 and WO 2015/118046 A1, respectively. A product which is a representative of such medium cut-off membranes and dialyzers and which is already available on the market today is the Theranova dialyzer (Baxter).

According to another aspect, the base membrane is a high cut-off membrane having a MWRO of from 15kDa to 20kDa and a MWCO of from 170kDa to 320kDa as measured by dextran sieving before blood contact. Such membranes are described, for example, in WO 2004/056460 A1 or in Gondouin and Hutchison: High Cut-Off Dialysis Membranes: Current Uses and Future Potential. Advances in Chronic Kidney Disease 18 (2011):180-187. A product which is a well-established representative of such high cut-off membranes and dialyzers, and which is available on the market today, is the Theralite dialyzer (Baxter). High cut-off membranes are characterized by a larger average pore size compared to the above-described medium cut-off membranes, whose mean pore size is lower and whose pore size distribution is narrower compared to standard high cut-off membranes.

According to another aspect, the base membrane is a high-flux (HF) membrane having an MWRO of from 5.0 kDa to 8.5 kDa and a MWCO of from 25 kDa to 50 kDa as measured by dextran sieving before blood contact.

According to yet another aspect of the invention, the membranes can be coated in two consecutive steps comprising (i) contacting a base membrane with a solution comprising chondroitin, and (ii) submitting the base membrane which is or has been in contact with a chondroitin solution to e-beam irradiation, thereby providing for a coated membrane comprising or essentially consisting of a base membrane which is coated with an essentially homogenous coating layer comprising or essentially consisting of chondroitin.

According to another embodiment of the invention, the base membrane is prepared from a polymer solution which comprises from 10 to 15 wt.%, relative to the total weight of the polymer solution, of polysulfone, polyethersulfone or polyarylethersulfone, and from 1 to 10 wt.%, relative to the total weight of the polymer solution, of polyvinylpyrrolidone.

According to another embodiment of the invention, a method or process for increasing the selectivity of a membrane is provided, wherein a base membrane is brought into contact with a solution comprising chondroitin and is subsequently submitted to e-beam irradiation at a dose of from 12.5 to 115 kGy, preferably a dose of from 20 to 100 kGy, either in the presence of the chondroitin solution or after discarding the chondroitin solution while the membrane is still wet.

According to another embodiment of the invention, the method is carried out with a base membrane comprising or essentially consisting of polysulfone (PS), polyethersulfone (PES), or polyarylethersulfone (PAES), and polyvinylpyrrolidone (PVP).

According to another embodiment of the invention, the method comprises e-beam irradiating the membrane in the presence of the chondroitin solution.

According to yet another embodiment of the invention, a process for the preparation a membrane is provided that comprises the steps of comprising the steps of

(a) Providing a base membrane, preferably a base membrane comprising, for example, a blend of a polysulfone (PS), polyethersulfone (PES) or polyarylethersulfone (PAES), and polyvinylpyrrolidone (PVP);
(b) Providing a chondroitin solution comprising chondroitin in an amount of from 5 mg/mL and 180 mg/mL;, preferably in an amount of from 10 mg/mL to 140 mg/mL;
(c) Contacting and optionally incubating the membrane of step (a) with the solution of step (b);
(d) Submitting the membrane of step (c) to e-beam irradiation at a dose of from 12.5 kGy to 115 kGy, preferably at a dose of from 20 kGy to 100 kGy, either in the presence of the chondroitin solution of step (b) or after having removed the chondroitin solution and while the membrane is still wet.

According to one embodiment of the invention, the lumen of a hollow fiber membrane (base membrane) is contacted with the solution of step (b) before and/or during e-beam irradiation. According to another embodiment, the outer surface of a hollow fiber base membrane is contacted with the solution of step (b) before and/or during e-beam irradiation. According to yet another embodiment of the invention, the complete accessible surface of a hollow fiber membrane is contacted with the chondroitin solution before or during e-beam irradiation. As will be understood by the skilled person, the solution may also enter the pores present on the lumen and/or outer surface of the membrane, at least to some extent. The surface of said pores will then be in contact and coated with chondroitin as well. It is also possible to submit the complete membrane to coating, wherein the lumen and outer surface as well the accessible surfaces of the pores on both surfaces of the membrane will be contacted with the coating solution.

According to another embodiment of the invention the membrane or the device comprising said membrane will be submitted to e-beam irradiation in the presence of the coating solution.

According to another aspect of the invention, the coating solution is removed after e-beam treatment, e.g., by emptying the device, optionally followed by one or more rinsing steps which can be carried out, for example, with water such as RO water. The membrane and device can then be processes as usual, i.e., it can be submitted to final sterilization, generally in a wet state (i.e., in the presence of residual water). Sterilization methods encompass e-beam or gamma sterilization according to methods known in the art. Alternatively, steam sterilization can be used.

According to one embodiment of the invention, a device is being provided which is a hemodialyzer or filter device which comprises a membrane coated with chondroitin according to the invention, wherein the membrane has an increased selectivity in comparison to the uncoated membrane. Such device is preferably for use in blood treatment applications, such as hemodialysis, hemofiltration, or hemodiafiltration applications.

**Brief Description of the Drawings**

Figure 1 is a schematic representation of sieving curves (e.g., dextran sieving curves) of hypothetic membranes having different selectivities as illustrated by their respective MWRO and MWCO values and the delta $\Delta$ between said MWRO and MWCO values. When the difference or delta ($\Delta$) between the molecular weight retention onset (MWRO) and molecular weight cut-off (MWCO) of a membrane decreases, the profile of the curve becomes steeper, i.e., the slope at the turning point increases, which corresponds to an increased selectivity of the membrane. Starting from sieving curve 1, this means that, for example, that the MWCO can remain the same or become lower (see MWCO2 and MWCO3, respectively), while the MWRO is shifted to higher molecular weights, resulting in MWRO2 or MWCO3. In consequence, the sieving curves 2 and 3 become steeper, i.e., the membrane becomes more selective.

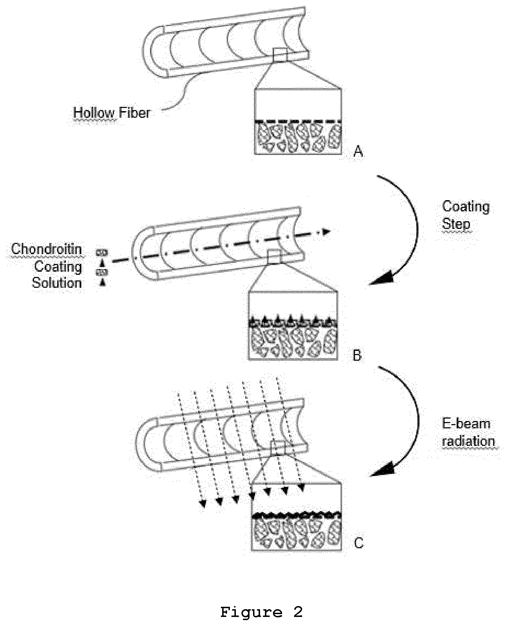

Figure 2 is a schematic representation of the process for coating the lumen of a base membrane with chondroitin. (A) shows a hollow fiber membrane ("base membrane") without coating. A coating solution comprising chondroitin is brought into contact with the lumen of the hollow fiber membrane in (B) for a given period of time which ranges from several minutes to several hours, wherein chondroitin gets into contact with the surface of the membrane. Preferably, the hollow fiber membrane is then submitted to e-beam irradiation in the presence of the coating solution, resulting in the immobilization of the chondroitin to the membrane surface.

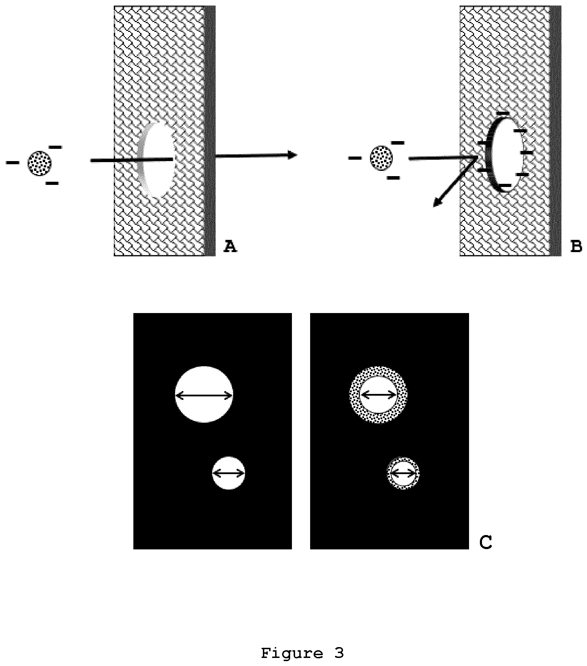

Figure 3 is a schematic representation of how the coating of the invention is hypothesized to improve the selectivity of a membrane. FIG. 3(A) shows an unmodified membrane, which is not charged. Negatively charged molecules such as, for example, albumin can pass through a membrane pore of appropriate size. FIG. 3(B) shows a membrane which is coated with negatively charged chondroitin whereby the membrane and pore receive a negatively charged layer and therefore reject passage of the same molecule which would have otherwise passed the pore. Another or additional effect that may contribute to an increase of rejection after modification is shown in FIG. 3(C). By reducing the pore size of larger pores more than smaller ones the selectivity of the overall membrane can also be increased.

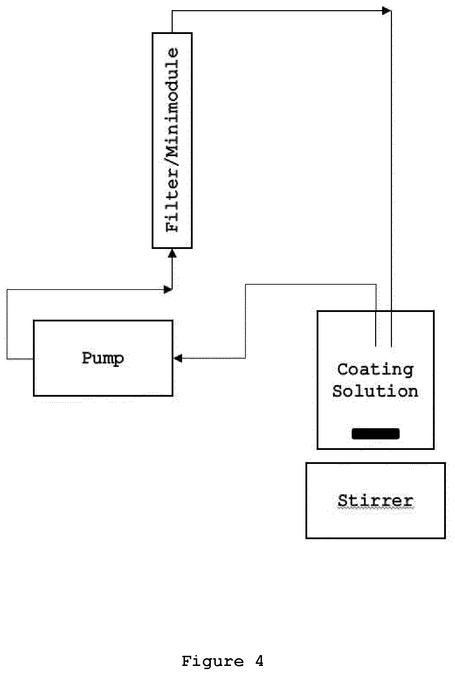

Figure 4 is a schematic representation of a process for coating hollow fiber membranes in a filter module, such as a minimodule, in recirculation mode, whereby the coating solution is pumped through the filter or minimodule. After a certain time, the pump can be stopped, and the filled filter (e.g., a hemodialyzer) or minimodule can be submitted to e-beam irradiation.

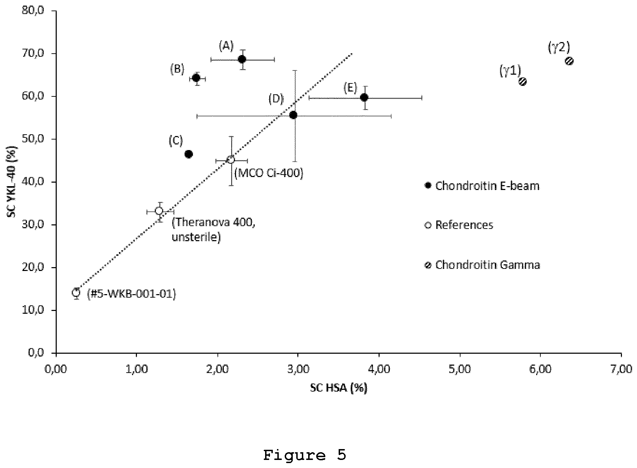

Figure 5 shows the effect of chondroitin + e-beam coating on sieving coefficients of human serum albumin (HSA) in % and YKL-40 in %. The Figure provides for a comparison with the uncoated membrane (MCO Ci-400), a commercially available medium cut-off membrane (Theranova 400, Baxter), and a standard high-flux membrane (#5-WKB-001-01). Said reference membranes are all shown with open circles. Filled circles depict membranes that have been treated with chondroitin and e-beam irradiation under various conditions. Hatched circles depict MCO Ci-400 base membranes with chondroitin but irradiated with gamma rays instead of e-beam ($\gamma$1 and $\gamma$2). All membranes have been contacted with a 20 mg/mL chondroitin solution. Membranes (in minimodules) A and B have been irradiated with 25 kGy and 75 kGy, respectively, both in the presence of the chondroitin solution ("filled"). Membrane (in minimodule) C was irradiated with 25 kGy in a "dry" state. Membranes D and E were irradiated with 75 kGy and 25 kGy, respectively, in the "wet" state, i.e., the chondroitin solution was discarded before submitting the membrane/module to e-beam irradiation. See **Table 6 and Table 7.** Theranova 400 was measured "unsterile" to be able to compare it with the coated membranes which have also been measured before final sterilization.

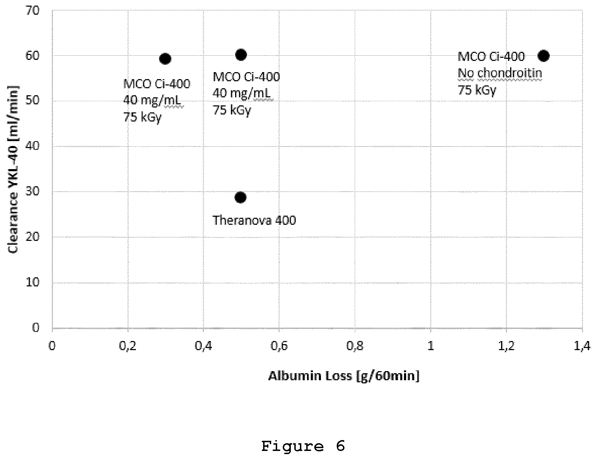

Figure 6 depicts the effects of the chondroitin/e-beam coating on albumin loss in g/60 min and YKL-40 clearance in ml/min. Albumin loss and clearance have been measured with standard filter modules (hemodialyzers) instead of minimodules. Theranova 400 was added as a reference membrane as a measure for selectivity of the membranes of the invention. MCO Ci-400 which was treated according to the protocols of the invention but in the absence of chondroitin in the solution was added for illustrating the impact of the coating. See **Table 9.** Figure 6 demonstrates that the coating of the invention significantly reduces albumin loss in comparison with the uncoated membrane but maintains the excellent clearance capabilities of YKL-40.

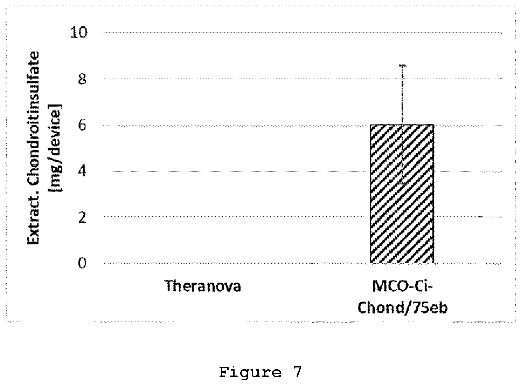

Figure 7 shows the amount of chondroitin which is eluted from a filter device (1.7 m$^2$) with ultrapure water in simulated treatment for 4h at 40°C (see **Example 6**). The filter device contained MCO Ci-400 hollow fiber membraned coated with a solution of 40 mg/mL chondroitin and e-beam irradiation with a dose of 75 kGy. Standard deviation: 2.6.

**Detailed Description**

The expression "selectivity" as used herein for a hemodialysis membrane refers to the membrane's ability to efficiently remove small (0.5-15kDa), medium (>15-25kDa) and large (>25-58kDa) middle-molecular weight compounds (see also Rosner et al.: Classification of Uremic Toxins and Their Role in Kidney Failure. Clin J Am Soc Nephrol. 2021) while essentially retaining essential, larger proteins such as, specifically, albumin (66.4 kDa). Such selectivity is described, for example, by the steepness of the sieving curve (e.g., dextran sieving curve) of a membrane. The increased steepness of the sieving curve correlates to increased selectivity, indicating that the membrane has an increased ability to remove large middle while efficiently retaining albumin. Accordingly, selectivity of a membrane can be described by its MWRO (molecular weight retention onset), i.e., the molecular weight at which the sieving coefficient of the membrane is 0.9, and its MWCO (molecular weight cut-off), i.e., the molecular weight at which the sieving coefficient of the membrane is 0.1, as these parameters provide for the necessary information on the shape and steepness of a membrane's sieving curve. For example, two membranes may have the same MWCO while differing in their MWRO. The membrane having the lower MWRO, accordingly, is less effective in removing molecules of a molecular weight below the cut-off. Its sieving curve is less steep, and the membrane is less selective than the one having a higher MWRO and the same MWCO. In other words, the selectivity of a membrane can be described by the delta $\Delta$ between its MWRO and MWCO. The smaller the delta $\Delta$ becomes, the higher the selectivity is. The steepness of the sieving curve is thus determined largely by the proximity of the values of the parameters MWRO and MWCO as visualized in Figure 1.

The expression "medium cut-off membrane(s)" or "medium cut-off dialyzer(s)" as used herein, which is sometimes interchangeably used with the expression "HRO membrane(s)" or "HRO dialyzer(s)" for "High Retention Onset" membrane(s) or dialyzer(s), refers to membranes and dialyzers such as described, for example, in WO 2015/118046 A1 and WO 2015/118046 A1, respectively, and wherein the membrane(s) preferably has (have) a MWRO of from 9 to 12.5 kDa and a MWCO of from 55 to 110 kDa as measured by dextran sieving before blood contact and as otherwise described in WO 2015/118046 A1. Preferably, medium cut-off membranes are further defined by a sieving coefficient for albumin ($Sc_{Alb}$(%)) of below 1 (<1) as measured in human plasma at QB=300ml/min and QUF=60ml/min; a sieving coefficient for $\beta$-2-microglobulin ($Sc_{\beta2m}$ (%)) of equal to or more than 95 ($\geq$95) as measured in human plasma at QB=300ml/min and QUF=60ml/min; and a sieving coefficient for YKL-40 ($Sc_{YKL-40}$ (%)) of equal to or more than 30 ($\geq$30) as measured in human plasma at QB=300ml/min and QUF=60ml/min. A medium cut-off dialyzer as mentioned herein is preferably further characterized by a KUF (ml/h/mmHg) of equal to or higher than 20 ($\geq$20) as measured according to ISO 8637; a $\beta$2m clearance $K_{\beta2m}$ (ml/min) of higher than 80 (>80) as measured according to ISO 8637 with QB=300-400mL/min; QD=700mL/min; QUF=0-10mL/min; a YKL-40 clearance $K_{YKL-40}$ (ml/min) of equal to or higher than 20 ($\geq$20) as determined in human plasma with QB=300ml/min; QD=500ml/min; QUF=10ml/min and preferably further has an albumin loss (g/4h in vivo treatment or simulated use correlated with in vivo use) of equal to or below 7 ($\leq$7.0), more preferably of equal to or below 4 ($\leq$4.0).

The expression "chondroitin" as used herein refers to chondroitin sulfate (CS), a polymer with a wide molecular weight range composed of an alternating sequence of sulfated and/or unsulfated d-glucuronic acid (GlcA) and N-acetyl- d-galactosamine (GalNAc) residues linked through alternating $\beta$-(1 → 3) and $\beta$-(1 → 4) bonds and derived from various sources, wherein the polymer can be sulfonated at various positions. The expression encompasses chondroitin sulfates which are classified as CS-O,A,B,C,D,E,F,K (CS-A, chondroitin sulfate A, [GlcA$\beta$1-3GalNAc(4 S)]; CS-C, chondroitin sulfate C, [GlcA$\beta$1-3GalNAc(6 S)]; CS-D, chondroitin sulfate D, [GlcA(2 S)$\beta$1-3GalNAc(6 S)]; CS-E, chondroitin sulfate E, , [GlcA$\beta$1-3GalNAc(4 S,6 S)]; CS-F, fucosylated chondroitin sulphate, [GlcA($\alpha$1-3Fuc)$\beta$1-3GalNAc(4 S)]; CS-O, chondroitin,[GlcA$\beta$1-3GalNAc]) according to their sulfation pattern, as well as CS extracted from different sources. The expression further pertains to CS which may consist of combinations of different types or classes of CS. If not expressly indicated otherwise, it preferably refers to the two major chondroitin sulfates CS-A and CS-C which differ from one another in the position of the sulfates. Chondroitin sulfate A is sulfated at position 4, and chondroitin sulfate C is sulfated at position 6.

The expression "base membrane" as used herein refers to a membrane before being coated with chondroitin according to the invention. Optionally, further compounds such as, for example, heparin may be present in the coating layer that is provided according to the present invention. In other words, the base membrane and the coated membrane differ regarding the presence of a coating layer comprising or essentially consisting of chondroitin. The base membrane may be altered during the coating process by the presence of a coating comprising or essentially consisting of chondroitin and changes to the base membrane due to the e-beam irradiation which is part of the coating process.

The expression "blood treatment" as used herein refers to the treatment of whole blood or any blood product, such as, for example, blood plasma, of a human or animal patient. Such treatment comprises, but is not limited to, hemodialysis, hemofiltration or hemodiafiltration.

The material of membranes used according to the invention may vary but will generally be uncharged membranes. Suitable base membranes in the context of the present invention comprise, for example, a blend of i) a polysulfone, a polyethersulfone or a polyarylethersulfone and ii) polyvinylpyrrolidone. Base membranes according to the invention can also be made from other known materials as the concept of selectivity increase as described herein is connected to the modification of the surface and pores of a given membrane. Other membrane materials that can be used are, for example, sulfonated polyacrylonitrile (PA or AN69), poly(methyl methacrylate) (PMMA), CTA (cellulose triacetate) or EVAL (ethylene vinyl alcohol copolymer). For an overview of membrane materials see, for example, Said et al., A Review of Commercial Developments and Recent Laboratory Research of Dialyzers and Membranes for Hemodialysis Application. Membranes 11, 767 (2021).

According to one aspect, base membranes according to the invention can have different physical structures, including varying degrees of asymmetric configuration, ranging from minimum asymmetry in sponge-like structures to maximum asymmetry in finger-like structures, such as described, for example, in Ronco and Clark, Nature Reviews. Nephrology 14 (6) (2018): 394-410). In case of hollow fiber membranes for use in hemodialysis, at least the lumen or inner side of the hollow fibers membranes is coated as it generally provides for the selective layer of the hollow fiber membranes that is in contact with blood, or, optionally, with blood plasma. In devices where the outer side of a hollow fiber membrane is in contact with blood and provides for the selective layer of the membrane, a modification or coating according to the invention would preferably be applied to the outer side of the hollow fiber membranes. It may be practical to coat the complete accessible surface of a membrane, including hollow fiber membranes, during the coating process. As will be understood by the skilled person, the accessible surface of pores on the lumen and/or outer surface of a hollow fiber may also be coated according to the invention.

According to one aspect, the base membrane is a medium cut-off membrane. A representative of such medium cut-off membranes which is available in the market today is the Theranova dialyzer (Baxter).

According to another aspect, the base membrane has a MWRO of from 8.5 to 14.0 kDa and a MWCO of from 50.0 to 130.0 kDa as measured by dextran sieving before blood contact, preferably a MWRO of from 8.5 to 12.5 kDa and a MWCO of from 55.0 to 110.0 kDa as measured by dextran sieving before blood contact. According to yet another aspect, the base membranes have a MWRO of from 9.0 to 12.5 kDa and a MWCO of from 55.0 or 60.0 to 90.0 kDa as measured by dextran sieving before blood contact.

According to another aspect, the base membrane has an MWRO of from 15kDa to 20kDa and a MWCO of from 170kDa to 320kDa as measured by dextran sieving before blood contact. Such membranes are described, for example, in WO 2004/056460 A1 or in Gondouin and Hutchison: High Cut-Off Dialysis Membranes: Current Uses and Future Potential. Advances in Chronic Kidney Disease 18 (2011):180-187. A representative of such high cut-off membranes available in the market today is the Theralite dialyzer (Baxter) which comprises such membrane.

According to another aspect, the base membrane is a high-flux (HF) membrane having an MWRO of from 5.0 kDa to 8.5 kDa and a MWCO of from 25 kDa to 50 kDa as measured by dextran sieving before blood contact. A representative of such high-flux membrane and dialyzer which is commercially available today is the Revaclear dialyzer (Baxter).

According to one aspect of the invention, the membranes can be coated in a two-step procedure, wherein the membrane or membrane surface is (i) contacted with a solution comprising or essentially consisting of chondroitin and (ii) subsequently submitted to e-beam irradiation. Generally, the coating step will be performed with the flat sheet membrane(s) or hollow fibers being provided or encased in a filter module according to the state of the art. E-beam irradiation will preferably be carried out while the coating solution is still present in the filter module. Alternatively, e-beam irradiation can be carried out after having emptied the filter module or container without drying of the membrane. Thus, e-beam irradiation can be carried out with "wet" membranes or membranes or modules containing at least residual solution. Another option includes the irradiation of membranes which have been rinsed once or several times, or the irradiation of modules which are filled with rinsing solution (e.g., water) after the chondroitin solution has been removed.

According to another embodiment of the invention, the coating step can be repeated once or several times. In this case, the coating solution is removed from the filter module and a fresh coating solution is added to the filter module and the hollow fibers. This process can be repeated several times. Generally, one, two, three, or four rounds of filling, incubating, and removing the coating solution can be carried out. Alternatively, the membrane can be contacted with the coating solution by recirculating the solution through the filter module such as shown, for example, in **Fig. 4.** Preferably, the coating solution remains in the filter module after the last incubation step or after a certain pumping time in case of recirculation, and the "filled" module is submitted to e-beam irradiation according to the invention.

According to one aspect, the solution for coating the base membrane is prepared, for example, by dissolving chondroitin in water (e.g., deionized water) or a buffer such as, for example, tris buffer. The solution can then be applied to the lumen of a hollow fiber membrane for the coating of the lumen of the membrane and any adjacent pore surfaces. For example, the coating solution can be filled into the lumen of the fibers by aspiration. Alternatively, the filter modules comprising a bundle of hollow fiber membranes can be filled with a syringe. The hollow fiber membrane can be incubated with the coating solution for a certain time which can be varied from several minutes to several hours or even days. Generally, five minutes to five hours of incubation or recirculation will be sufficient depending on the base membrane and the concentration of the chondroitin in the solution. The lumen of the hollow fiber can optionally be rinsed after incubation, e.g., with 0.9% NaCl or distilled water. The flow rate for filling and/or rinsing of a hollow fiber membrane module can be varied over a relatively broad range, e.g., from about 10 mL/min to 50 ml/min, and the rinsing time can also be varied from about 1 to 20 minutes, such as, for example, from about 2 to 10 minutes.

As mentioned above, hollow fiber membranes can be submitted to the initial step of coating in recirculation mode, wherein the coating solution is provided in a pool from which it is pumped, for example, through the hollow fibers of a filter device (**Fig. 4**). The pool volume can vary, but about 100 to 500 mL of a coating solution will be sufficient for an effective initial coating of a standard dialyzer having a surface area of about 1.5 -2.2 $m^2$. The pump flow can also be varied over a relatively broad range, such as, for example, from 1 mL/min to about 20 mL/min. After coating the filters can be rinsed as described before or directly be submitted to e-beam irradiation in a filled state.

According to yet another aspect of the invention the concentration of chondroitin in the coating solution is between 5 mg/mL and 160 mg/mL, preferably between 10 mg/mL and 120 mg/mL, such as, for example, between 10 mg/mL and 100 mg/mL, or between 10 mg/mL and 80 mg/mL, including concentrations of 20 mg/mL, 30 mg/mL, 40 mg/mL, 50 mg/mL, 60 mg/mL, 70 mg/mL or 80 mg/mL.

According to one aspect of the invention, chondroitin sulfate A (CS-A) is used for coating the membranes. Chondroitin sulfate A is available in different molecular weights ranging from, for example, 4 kDa to 50 kDa. According to the invention, all chondroitin molecular weight variants can be used for the coating of a membrane. According to one embodiment of the invention, chondroitin with a higher molecular weight, such as from about 10 kDa to 50 kDa, from 20 kDa to 50 kDa, or from 20 kDa to 30 kDa is used for coating a membrane. According to yet another aspect of the invention, chondroitin with a lower molecular weight, such from about 1 kDa to 15 kDa, such as from 4 kDa to 10 kDa, is used for coating a membrane. As mentioned before, also mixtures of different CS variants can be used, e.g., mixtures of CS-A and CS-C. Mixtures that predominantly contain CS-A are preferably used according to the inventions as it was found that they are specifically well able to increase the selectivity of a membrane. However, also CS containing predominantly CS-C can be used, as well as various combination of CS-A and CS-C.

According to another aspect of the invention, the membranes are coated with chondroitin in an amount of from 0.30 $\mu$g/cm$^2$ to 2.00 $\mu$g/cm$^2$, such as 0.30 $\mu$g/cm$^2$, 0.40 $\mu$g/cm$^2$, 0.50 $\mu$g/cm$^2$, 0.60 $\mu$g/cm$^2$, 0.70 $\mu$g/cm$^2$, 0.80 $\mu$g/cm$^2$, 0.90 $\mu$g/cm$^2$, 1.00 $\mu$g/cm$^2$, 1.10 $\mu$g/cm$^2$, 1.20 $\mu$g/cm$^2$, 1.30 $\mu$g/cm$^2$, 1.40 $\mu$g/cm$^2$, 1.50 $\mu$g/cm$^2$, 1.60 $\mu$g/cm$^2$, or 1.70 $\mu$g/cm$^2$,. According to yet another aspect of the invention, the membranes are coated with chondroitin in an amount of from 0.20 $\mu$g/cm$^2$ to 1.20 $\mu$g/cm$^2$.

The presence and amount of chondroitin on the finished fibers can be tested with known assays such as the dimethylmethylene blue (DMMB) assay (Farndale et al., Biochimica et Biophysica Acta 883: 173-177 (1986)).

The membranes and filters, respectively, are submitted to e-beam irradiation according to the invention. E-beam irradiation is carried out according to know procedures, preferably at a dose of from 12.5 kGy to 110 kGy, such as from 12.5 to 90 kGy, from 12.5 to 80 kGy, from 20 to 90 kGy, from 30 to 90 kGy, or from 50 to 100 kGy, for example, at 12.5 kGy, 15 kGy, 20 kGy, 25 kGy, 30 kGy, 35 kGy, 40 kGy, 45 kGy, 50 kGy, 55 kGy, 60 kGy, 65 kGy, 70 kGy, 75 kGy, 80 kGy, 85 kGy, 90 kGy, 95 kGy, or 100 kGy.

As mentioned before, it may be preferable to submit a membrane, hollow fiber bundle, or filter device comprising such membrane or hollow fiber bundle to e-beam irradiation in the presence of the coating solution which comprises or essentially consist of chondroitin according to the invention. This is referred to herein as the "filled" state, as in the case of using a filter device or minimodule which comprises, for example, hollow fiber membranes, the device is "filled" with the coating solution and submitted to irradiation according to the invention. After the irradiation process, the coating solution is removed, e.g., by emptying the filter device, optionally followed by blowing out the device and/or rinsing the device and hollow fibers with water, such as RO water. Optionally, the membranes are dried before sterilization. The membrane and device have to undergo final sterilization after the coating procedure. Final sterilization is done by known methods, such as, for example, e-beam sterilization at 25 kGy or gamma irradiation. Steam sterilization is also possible but may have a more pronounced impact on the coating of the membrane.

According to one aspect of the invention, the above-described modification and/or coating of a given membrane, such as a polysulfone, polyarylethersulfone or polyethersulfone based membrane, with chondroitin in combination with e-beam irradiation, has an unexpected influence on the sieving performance of and, in consequence, on the selectivity of the membrane. Surprisingly, the combination of chondroitin coating and e-beam irradiation shows excellent effects on the selectivity of a membrane. Accordingly, the present invention provides for an improved selectivity of the treated membranes while at the same time the post-spinning modification of the membrane and medical device comprising such membrane is not very complex. For example, compared to earlier approaches using (poly)dopamine as an intermediate in the coating of membranes, the current approach has the benefit of a simplified modification procedure, no leaching of (poly)dopamine, and an improved YKL-40 clearance combined with a reduced albumin loss.

Marker compounds that can be used for assessing said influence on selectivity by determining their respective sieving coefficients are advantageously selected from medium to large middle molecules, such as, for example, YKL-40. In addition, albumin is advantageously used as another or second marker molecule. For determining the effect of the coating according to the invention on the sieving performance and selectivity of a given base membrane, sieving coefficients for the selected marker molecules can be determined before and after the modification with chondroitin/e-beam.

As explained with a view on MWRO and MWCO and the delta $\Delta$ between same (see **Fig. 1**), a similar assessment can be made based on dextran sieving.

According to another aspect of the invention, the modified or coated membranes according to the invention retain their hemocompatibility also after coating and final sterilization. According to one aspect of the invention, the membranes coated according to the invention can be used as membranes in medical applications, including for applications that require a direct contact of the coated surface with the blood or blood components of a patient. For example, coated membranes according to the invention can be used for preparing dialyzers for use in hemodialysis, including chronic and acute (CRRT) applications and encompassing hemodiafiltration and hemofiltration.

According to yet another aspect of the invention, filters or hemodialyzers are provided that comprise hollow fiber membranes that have been coated according to the invention on the lumen side, the outer surface, or all accessible surfaces of the hollow fiber membranes. According to one aspect, said filters or hemodialyzers comprise hollow fiber membranes which are coated with chondroitin, or, in other words, which have a coating layer that comprises or essentially consists of chondroitin.

According to another aspect of the invention, the filters and dialyzers of the invention that comprise hollow fiber membranes which are modified and/or coated with chondroitin according to the invention can be used in extracorporeal blood purification applications or extracorporeal blood treatment applications, including, for example, hemodialysis, hemodiafiltration and hemofiltration, including CRRT.

It will be readily apparent to one skilled in the art that various substitutions and modifications may be made to the invention disclosed herein without departing from the scope and spirit of the invention.

The present invention will now be illustrated by way of non-limiting examples to further facilitate the understanding of the invention.

**Examples**

**Example 1: Basic Materials and Methods**

**Example 1.1: Preparation of minimodules**

Minimodules (fibers in a housing used as standardized filters for analysis) are prepared by cutting fibers to a length of 20 cm, drying the fibers for 1 h at 40°C and <100 mbar and subsequently transferring the fibers into the housing. The ends of the fibers are potted with polyurethane. After the polyurethane has hardened, the ends of the potted membrane bundle are cut to reopen the fibers. The minimodule ensures protection and adequate presentation of the fibers. In the present examples, minimodules having an effective membrane (lumen) surface A of 360 cm$^2$ were used. The number of fibers required for an effective surface A of 360 cm$^2$ is calculated according to equation (1)

$$A = \pi \times d_i \times l \times n \, [cm2] \qquad (1),$$

wherein $d_i$ is the inner diameter of fiber [cm], n represents the number of fibers, and 1 represents the effective fiber length [cm]. Each minimodule has an effective length of approx. 170 mm (without PU potting) and an inner diameter of 10 mm. The internal diameter of fibers and the wall thickness depends on the specific membranes used. Hence, the packing density generally varies between 23% and 31%.

**Example 1.2: Hydraulic Permeability (Lp) of minimodules**

The hydraulic permeability of a minimodule is determined by pressing a defined volume of water under pressure through the minimodule, which has been sealed on one side, and measuring the required time. The hydraulic permeability is calculated from the determined time t, the effective membrane surface area A, the applied pressure p and the volume of water pressed through the membrane V, according to equation (2):

$$Lp = V / [p \cdot A \cdot t] \quad (2)$$

The effective membrane surface area A is calculated from the fiber length and the inner diameter of the fiber according to equation (3)

$$A = \pi \cdot d_i \cdot l \cdot [cm^2] \quad (3)$$

wherein $d_i$ represents the inner diameter of fiber [cm] and 1 represents the effective fiber length [cm].

The mini-module is wetted thirty minutes before the Lp-test is performed. For this purpose, the minimodule is put in a box containing 500 mL of ultrapure water. After 30 minutes, the minimodule is transferred into the testing system. The testing system consists of a water bath that is maintained at 37°C and a device where the minimodule can be mounted. The filling height of the water bath must ensure that the minimodule is located underneath the water surface in the designated device. In order to avoid that a leakage of the membrane leads to a wrong test result, an integrity test of the minimodule and the test system is carried out in advance. The integrity test is performed by pressing air through the minimodule that is closed on one side. Air bubbles indicate a leakage of the minimodule or the test device. It must be checked if the leakage is due to an incorrect mounting of the minimodule in the test device or if the membrane leaks. The minimodule must be discarded if a leakage of the membrane is detected. The pressure applied in the integrity test must be at least the same value as the pressure applied during the determination of the hydraulic permeability in order to ensure that no leakage can occur during the measurement of the hydraulic permeability because the pressure applied is too high.

**Example 1.3: Dextran sieving measurements**

**Example 1.3.1: Dextran solutions**

Fractions of dextran supplied by Fluka (Mw 6, 15-20, 40, 70, 100, 200, 500 kDa) and Sigma-Aldrich (Mw 9-11 kDa) (both from Sigma-Aldrich Co. LLC, St. Louis, USA) are used without further purification. Solutions of dextrans with the different molecular weight fractions are combined in Millipore water (i.e., Type 1 ultrapure water, as defined by ISO 3696) at a concentration of 1 g/l for each fraction, which results in an overall concentration of 8 g/l.

**Example 1.3.2: Dextran sieving coefficient tests**

Filtration experiments are carried out under a constant shear rate ($\gamma$=750s$^{-1}$) and with the ultrafiltration rate set at 20% of the blood side entrance flux $Q_{Bin}$, calculated according to equation (4):

$$Q_{Bin} = \frac{\gamma \cdot n \cdot \pi \cdot d_i^3 \cdot 60}{32} \quad (4),$$

where $Q_{Bin}$ is the flux at the blood side entrance in ml/min; n is the number of fibers in the minimodule; $d_i$ is the inner diameter of the fibers in cm and $\gamma$ is the constant shear rate mentioned above. The filtration conditions are without backfiltration, contrary to the conditions typical of hemodialysis. The dextran solution is recirculated at 37°C $\pm$ 1°C. Feed (blood side entrance), retentate (blood side exit), and filtrate (dialysate exit) samples are taken after 15 min. Relative concentrations and molecular weights of the samples are analyzed via gel permeation chromatography. The analysis is carried out in a High Performance Liquid Chromatography (HPLC) device (HP 1090A or Agilent 1200; Agilent, Santa Clara, CA, USA) equipped with an RI detector (G1362 from Agilent) and TSKgel columns (PWXL-Guard Column, G 3000 PWXL, G 4000 PWXL; Tosoh, Tessenderlo, Belgium). Samples are filtered through a 0.45 $\mu$m filter type OE67 from Schleicher and Schnell, Einbeck, Germany. Calibration is done against dextran standards (Fluka). The sieving coefficient SC is calculated according to equation (5)

$$SC = \frac{2 \cdot c_F}{c_P + c_R} \quad (5),$$

wherein $c_F$ is the concentration of the solute in the filtrate, $c_P$ its concentration in the permeate and $c_R$ its concentration in the retentate.

**Example 1.4: Measurement of sieving coefficients in human plasma**

Middle molecules, consisting mostly of peptides and small proteins with molecular weights in the range of 500-60,000 Da, accumulate in renal failure and contribute to the uremic toxic state. Beta2-microglobulin (beta2-MG or $\beta$2-M) with a molecular weight of 11 kDa is considered one of the smaller representatives of these middle molecules. Myoglobin has a molecular weight (MW) of about 17 kDa is already larger. It will not be completely cleared from blood by known high-flux dialyzers, whereas it is readily removed by medium cut-off or high cut-off dialyzers. YKL-40 with a molecular weight of 40 kDa is considered a representative of higher molecular weight middle molecules that should still be removed as efficiently as possible. Albumin with a MW of about 67 kDa is a key element in describing the sieving characteristics of membranes, as albumin should preferably not be allowed to pass a membrane for chronic hemodialysis to a significant extent, whereas molecules having a lower molecular weight, such as $\beta$2-M and YKL-40 should be removed as efficiently as possible, i.e., the sieving curve of a given membrane should be steep and not flat, as is typically found with protein-leaking membranes.

The sieving coefficients for albumin and YKL-40 which are naturally contained in human plasma can be determined for uncoated membranes and membranes that were coated according to the invention or of comparative examples. Determination of sieving coefficients for these marker molecules can be done, for example, with Octaplas LG from Octapharma.

For the experiments, human plasma is thawed and brought to 37°C under careful stirring. Other marker substances can be added to the plasma as required. For each minimodule 150g of human plasma is used. The final protein concentration of the test solution is 4515 g/l. The minimodules are rinsed with 40 mL 0.9% NaCl solution on the blood and dialysate side. Then the blood and dialysate sides are blown out at $0.3 \pm 0.1$ bar for $10 \pm 5$ s. $Q_B$ was 8,7 mL/min (= 7,71 SKT) and $Q_{UF}$ was 1,7 mL/min (=1,58 SKT) . The minimodules are connected to a new tubing set with the open filtrate side on top. The recirculating tubes are returned to the solution reservoir. The blood-in pump is started. When the minimodules are free of air bubbles, the filtrate pump is started. As soon as filtrate flows back from the tube into the pool time is started. After some time a sample of the test solution (A) (minimum 1,1 mL for HSA and 550 $\mu$L for YKL-40) can be taken. The sampling time is 1 min for $Q_{UF}$ and 1 min for $Q_{Bout}$. From these measurements, the retentate sample (R) and filtrate sample (F) are used for further analysis of the marker molecules. The sieving coefficient SC is calculated according to equation (5).

**Example 1.5: Clearance Performance**

The clearance "C" (mL/min) refers to the volume of a solution from which a solute is completely removed per unit time. In contrast to the sieving coefficient which is the best way to describe a membrane as the essential component of a hemodialyzer, clearance is a measure of the overall dialyzer function and hence dialysis effectiveness. Accordingly, the coating as described before was also applied to complete hemodialyzers, such as commercially available dialyzers like Theranova 400 (Baxter Int., Inc.) or hemodialyzers were prepared from other MCO type membranes, such as the MCO-Ci 400 dialyzer (Gambro Dialysatoren GmbH, Hechingen, Germany); see, for example, Boschetti-de-Fierro et al. (2015), Scientific Reports 5: 18448, DOI: 10.1038/srep18448) and were coated according to the invention. If not indicated otherwise, the clearance performance of a dialyzer was determined according to ISO 8637:2004(E). The set-up of the test circuit was as shown in ISO 8637:2004(E). Flows are operated in single path.

To measure clearance, human plasma (protein content $55 \pm 10$ g/L) is spiked with myoglobin and $\beta$2-M (Lee Biosolutions), to a concentration of 0.50 mg/L and 5.0 mg/L, respectively. Albumin and YKL-40, for example, are measured directly from plasma (e.g., OctaplasLG 45-70 mg/mL from Octapharma). The test is performed by recirculating human plasma for $60 \pm 5$ minutes at QB = 300mL/min, QD = 500mL/min, and UF = 10 mL/min at a temperature of 37°C. Samples are in each case taken from the blood outlet, dialysate outlet and blood inlet, respectively. Samples are taken at t [min]= 0, 4, 10, 20, 30, 40, 50, 60. Clearance (Cl) is calculated according to equation (6)

$$c(t) = c_0 \cdot e^{-t \cdot \frac{Cl}{V(t)}} \qquad (6),$$

wherein c(t) is the concentration of the marker (e.g., YKL-40) protein at time (t), V is the plasma volume and co is the concentration of the marker at t=0.

**Example 1.6: Quantitative determination of YKL-40 in human plasma**

For determining the concentration of YKL-40 the Quantikine Human Chitinase 3-like (CHI3L1) is used according to the supplier's instructions. The assay is a solid phase ELISA assay designed to measure CHI3L1 in cell culture supernates, serum, plasma, and urine. The assay employs the quantitative sandwich enzyme immunoassay technique. A monoclonal antibody specific for the analyte has been pre-coated onto a microplate. Standards and samples are pipetted into the wells and any analyte present is bound by the immobilized antibody. After washing away any unbound substances, an enzyme-linked polyclonal antibody specific for the analyte is added to the wells. Following a wash to remove any unbound antibody-enzyme reagent, a substrate solution is added to the wells and color develops in proportion to the amount of CHI3L1 in the initial step. The color development is stopped, and the intensity of the color is measured.

For determining the concentration of β2-M, myoglobin and albumin a nephelometer (BN ProSpec, Siemens Medical Solutions) is used in combination with the supplier's quantification kits. β2-M, myoglobin and albumin are quantified using the N Latex β2 Microglobulin kit, N Latex Myoglobin kit and N Antiserum to Human albumin kit (Siemens Health solutions), respectively, all according to the supplier's instructions. Each determinant is measured separately by formation of an insoluble antigen-antibody complex upon addition of the provided antibody. The complex formation proceeds in excess of antibody, is measured using light scattering by the nephelometer and compared to standards of known concentration.

**Example 1.7: Determination of albumin loss**

Albumin loss is determined in the same setup as described for the determination of clearance (**Example 1.5**). Samples are taken at the dialysate outlet at t [min] = 4, 10, 20, 30, 40, 50, 60. Albumin concentration can be determined by spectrophotometry according to known protocols, see also **Example 1.6.**

**Example 2: Coating of hollow fiber membranes with chondroitin**

A solution of chondroitin A sulfate sodium salt with a high molecular weight and an average MW of 10,000 to 50,000 Da from Biosynth was used for the experiments if not indicated otherwise. Solutions of other chondroitin sulfate types were prepared the same way. 300 mL of coating solution were used per minimodule (see **Example 1.1**). The solutions used for coating had final concentrations of from 20 mg/mL to 160 mg/mL as indicated.

The chondroitin solutions were prepared by stirring until the chondroitin was dissolved completely, which takes longer at higher chondroitin concentrations. The resulting solutions were immediately used for the coating process. For example, the lumen and pores of the fibers of a minimodule can be filled with the chondroitin solution by suction, wherein the upper connector that is fluidly connected to the lumen (blood side) of the hollow fibers is closed, and the bottom connector that is fluidly connected to the blood side of the fibers or module is connected to the coating solution and then closed as well. The connector at the bottom of the module that is fluidly connected to the filtrate or dialysate side of the module is also closed, whereas the respective (dialysate side) connector at the upper side of the module is connected to a vacuum pump. About 40 mL of the coating solution are pumped into the filter module with the help of a vacuum pump, wherein the blood side connector at the bottom of the module is opened after a vacuum has been achieved. The coating solution is thus sucked through the lumen side of the membrane to the outside of the membrane, resulting in a complete coating of the membrane surfaces, including the pores. After the coating solution is completely sucked in, the module is left standing for a short while, then all connectors are closed, the module is disconnected and sent to irradiation. During the filling process the minimodule can optionally be shaken to remove air bubbles.

Alternatively, the hollow fibers are coated by connecting the module to a peristaltic pump ("filling"). Tubes are connected to the blood side of the module whereas the connectors being in fluid communication with the dialysate side of the module are closed. The coating solution is pumped into the module with about 100 mL/min until the complete solution has been transferred into the module. Again, the formation of air bubbles is avoided. All connectors are then closed, the module is disconnected and prepared for irradiation.

The chondroitin coating solution can also be filled into the minimodules by using a 50 mL syringe filled with about 5 mL of the chondroitin solution. Again, minimodules were closed and left for 15 minutes, after which 50 mL syringes filled with air were used to empty them. The emptied "wet" minimodules were again closed and sent to e-beam irradiation. Alternatively, the "filled" minimodules still containing the chondroitin solution were closed without emptying them and sent to e-beam irradiation.

The initial coating with chondroitin can equally be performed with standard size hemodialyzers, wherein, for example, about 100 mL of the coating solution are used instead of 5 mL as for the minimodules.

Known hollow fiber membranes such as otherwise used in commercial hemodialyzers (Theranova 400, Baxter) or in experimental membranes which have been described before, such as MCO-Ci 400 (see, for example, Boschetti-de-Fierro et al., Scientific Reports (2015) 5: 18448, wherein "MCO 4" corresponds to MCO-Ci 400, or WO 2015/118045 A1), all from Baxter, were used for preparing minimodules according to **Example 1.1** or filter devices and coated as described above in this **Example 2.** An overview over certain minimodules prepared according to the invention and used in subsequent tests is provided in **Table 1.**

**Table 1: Minimodules prepared according to the invention.**

| # | Membrane | Chondroitin concentration (mg/mL in water) | Filter Preparation | E-beam dose [kGy] | Designation of Test Module |
|---|----------|-------------|-------|------|--------|
| 1 | **MCO Ci 400** | **20** | **Filled** | **75** | **MM3 6** |
| 2 | **MCO Ci 400** | **20** | **Filled** | **75** | **MM4 3** |
| 3 | **MCO Ci 400** | **20** | **Filled** | **75** | **MM22** |
| 4 | MCO Ci 400 | 20 | Wet | 75 | MM33 |
| 5 | MCO Ci 400 | 20 | Wet | 75 | MM35 |
| 6 | MCO Ci 400 | 20 | Wet | 75 | MM44 |
| 7 | MCO Ci 400 | 20 | Wet | 25 | MM31 |
| 8 | MCO Ci 400 | 20 | Wet | 25 | MM34 |
| 9 | MCO Ci 400 | 20 | Wet | 25 | MM37 |

**Example 3: Quantification of chondroitin**

The amount of chondroitin on polyethersulfone (PES) fibers after coating can be determined by placing fibers in 2 mL cryovials and adding the respective detection reagents for each substance. For quantifying chondroitin, a dimethylmethylene blue (DMMB) assay was used (Farndale et al., Biochimica et Biophysica Acta (1986) 883: 173-177).

The tests were carried out by using a 200 $\mu$g/mL stock solution, of which 200 $\mu$l were combined with 2mL of reagent. Since solid membranes were used, the dilution of the stock solution in the reagent was utilized to calculate the final standard concentration and this number was then used to determine the respective quantities present on the membranes.

**Example 4: E-beam irradiation and dose**

E-beam irradiation was done according to known methods. The coated membranes in minimodules or filter modules were submitted to e-beam irradiation with focused electrons (beta particles) at various doses. E-beam irradiation was typically performed at 25 or 75 kGy. The process was performed with "dry", "wet" and "filled" devices (see also **Example 2**) when being submitted to e-beam irradiation.

**Example 5: Influence of process parameters on sieving coefficients, clearance and albumin loss**

**Example 5.1: Influence of e-beam dose and chondroitin concentration**

Experiments based on MCO Ci-400 minimodules as described before were performed to determine the influence of e-beam dose on the selectivity of the membrane. As a measure for selectivity, the sieving coefficients of YKL-40 and HSA were determined. The minimodules were irradiated in the "filled" state to make them comparable and several minimodules were tested for each value as indicated. **Table 2** summarizes the outcome of the coating experiments with different doses.

Table 2: MCO Ci-400 membranes in minimodules were submitted to different chondroitin concentrations and doses to test the influence of both parameters on the increase of selectivity of the base membrane after coating as determined by sieving coefficients for HSA (Human Serum Albumin) and YKL-40. Tests were carried out as described herein with human plasma. Lp is provided in [*$10^{-4}$ cm /(bar*s)]. "MV" refers to "Mean Value", "SD" to "Standard Deviation". "n" designates the number of minimodules/ test performed. Chondroitin concentrations having an asterisk (*) mean that chondroitin from Mythocondro was used (CS-C, MW of 1-5 kDa), whereas all other coatings were done with chondroitin from Biosynth (CS-A, MW of 10-30 kDa).

| # | Coating with chondroitin | Irradiation Conditions | LP (MV) | n | Sieving Coefficient HSA | | | |
|---|---|---|---|---|---|---|---|---|
| | | | | | HSA [%], 30 min. | | YKL-40 [%], 30 min. | |
| | | | | | MV | SD | MV | SD |
| 1 | 20 mg/mL (filled) | **75 kGy** | 175 | 4 | **1,75** | 0, 10 | **64,0** | 1, 49 |
| 2 | 20 mg/mL (filled) | **75 kGy** | 150 | 3 | **1,83** | 0,21 | **63,7** | 0, 02 |
| 3 | 20 mg/mL (filled) | 25 kGy | 178 | 3 | 2,32 | 0,39 | 68,5 | 2,38 |
| 4 | 20 mg/mL (filled) | 25 kGy | 425 | 4 | 6,37 | 0,77 | 67,9 | 2,45 |
| 5 | 40 mg/mL (filled) | 25 kGy | 147 | 4 | 1,82 | 0, 02 | 78,2 | 5, 89 |
| 6 | 40 mg/mL (filled) | **75 kGy** | 138 | 4 | **1,01** | 0,34 | **76,4** | 4,22 |
| 7 | 40 mg/mL (filled) | **75 kGy** | 118 | 4 | **1,05** | 0, 13 | **61,9** | 7,75 |
| 8 | 40 mg/mL (filled) | **75 kGy** | 154 | 4 | **1,17** | 0, 08 | **57,9** | 3, 70 |
| 9 | 40 mg/m (filled) | **75 kGy** | 113 | 4 | **0,37** | 0, 03 | **45,0** | 1, 97 |
| 10 | 40 mg/mL* (filled) | **75 kGy** | 200 | 4 | **2,40** | 0,29 | **67,0** | 2,28 |
| 11 | 80 mg/mL* (filled) | **75 kGy** | 138 | 4 | tbd | tbd | tbd | tbd |
| 12 | 120 mg/mL* (filled) | **75 kGy** | 134 | 4 | tbd | tbd | tbd | tbd |
| 13 | 160 mg/mL* (filled) | **75 kGy** | 143 | 4 | tbd | tbd | tbd | tbd |
| 14 | No coating, unsterile | - | 193 | 2 | 1.8 | - | 38.9 | - |

As can be seen in **Table 2,** higher e-beam doses (75 kGy versus 25 kGy) result in an increased improvement of selectivity versus the uncoated base membrane MCO Ci-400 (see **Table 5** for comparison with the uncoated base membrane), even though both doses are effective in improving membrane selectivity. Comparing the chondroitin concentrations used, the data further show that higher chondroitin concentrations, such as, for example 40 mg/mL in comparison to 20 mg/mL (e.g., #4/5 or #2/6), enhance the selectivity increase.

**Example 5.2: Hydraulic permeability and the influence of the coating process**

**Table 3** provides for the hydraulic permeability of membranes according to the invention. Tests were performed as described in **Example 1.2** on minimodules shown in **Table 1** at same chondroitin concentrations for better comparability. The results confirm that the Lp values are within expected ranges, indicating that the coating according to the invention does not negatively affect hydraulic permeability of the membranes, which is an important prerequisite for the applicability of the membranes and methods of the invention in the field of dialysis. Lp values for the minimodules submitted to e-beam irradiation in a filled state (shown in bold, 36, 43, 22) showed a lower Lp compared to those that were irradiated in a wet state, indicating that membrane coating is different depending on the process used and indicating that irradiation in the filled state might be preferable.

Table 3: Lp measurements for membranes/minimodules coated and irradiated according to the invention with chondroitin.

| Mini-module (MM) | Lp-Wert [$10^{-4}$ cm/s*bar] | Fiber diameter [$\mu$m] | Number of fibers | Fiber length [cm] | Membrane surface [$cm^2$] |
|---|---|---|---|---|---|
| **36** | **162,8** | **180** | **371** | **12,9** | **271** |

(continued)

| Mini-module (MM) | Lp-Wert [$10^{-4}$ cm/s*bar] | Fiber diameter [$\mu$m] | Number of fibers | Fiber length [cm] | Membrane surface [$cm^2$] |
|---|---|---|---|---|---|
| **43** | **171,0** | **180** | **375** | **12,9** | **274** |
| **22** | **174,0** | **180** | **373** | **13,0** | **274** |
| 33 | 389, 8 | 180 | 375 | 13,1 | 278 |
| 35 | 305, 8 | 180 | 374 | 13,1 | 277 |
| 44 | 337,7 | 180 | 370 | 12,9 | 270 |
| 31 | 291,4 | 180 | 374 | 13,0 | 275 |
| 34 | 297,0 | 180 | 375 | 13,0 | 276 |
| 37 | 233,6 | 180 | 375 | 12, 9 | 274 |

**Example 5.2: Sieving coefficients and the influence of the coating process**

Sieving coefficients (SC) for albumin and (HAS) and YKL-40 were determined for membranes according to the invention as described in **Example 1.4.** The focus in this experiment was on the influence of the irradiation process. Accordingly, minimodules in a filled state (bold) were tested in comparison with those in a wet state. The results are provided in **Table 4.**

**Table 4: Sieving coefficients for human serum albumin (HAS) and YKL-40. Tested membranes (in minimodules) were as described in Table 1. Membranes and minimodules shown in bold are those which were irradiated in the "filled" state.**

| Minimodule (MM) | Conc. HSA [g/L] | SC HSA [%] | Conc. YKL-40 [pg{mL] | SC YKL-40 [%] |
|---|---|---|---|---|
| **36** | **31.9** | **1.82** | **20832.2** | **62.49** |
| **43** | **32,4** | **1,64** | **21307,9** | **64,02** |
| **22** | **31,1** | **1,79** | **21457,1** | **65,46** |
| 33 | 32,0 | 4,60 | 22963 | 60,01 |
| 35 | 32,7 | 3,26 | 20950,8 | 62,08 |
| 44 | 31,7 | 3,63 | 21397,4 | 56,59 |
| 31 | 32,2 | 3,65 | 20035,3 | 61,34 |
| 34 | 32,1 | 3,64 | 21069,7 | 61,64 |

The results provided in **Table 4** show that the coating of the base membrane with chondroitin followed by e-beam irradiation leads to change of the sieving coefficients for HSA and YKL-40 of the coated membrane compared to the uncoated membrane. The data further show that the results are different for the different methods used for producing the coated membranes. Those modules that were subjected to e-beam irradiation in a filled state (coating solution is left in the modules during irradiation) show a somewhat higher selectivity, i.e., the sieving coefficients for HSA are lower in comparison to those membranes and modules that were irradiated in the "wet" state. As a result, the sieving curve for those membranes that were irradiated in a filled module are steeper than those irradiated in a wet state. It is therefore preferable to submit the membrane or filter modules to e-beam radiation in the "filled" state.

The impact of coating on the sieving coefficients for human serum albumin (HSA) and YKL-40 and on the selectivity of the membrane can be determined by comparing uncoated membranes with the same membranes after having been treated according to the invention. **Table 5** provides for the hydraulic permeability as well as sieving coefficients for HSA and YKL-40 for reference membrane MCO-Ci 400 (uncoated), which is the same membrane that was used in the coating experiments shown before. In addition, **Table 5** also shows Lp and SC values measured with a high-flux type membrane (#5-WBK-001-01, unsterile) which is included for comparison of Lp and SC values, and the Theranova 400 membrane (also unsterile for better comparability).

**Table 5: Lp and SC for the uncoated MCO-Ci-400 membrane. Also shown are the Theranova membrane (unsterile), and #5-WKB-001-01, which is a high-flux type membrane. The values shown are average values based on (9), (4), and (9) measurements, respectively.**

| Membrane | Lp [E-4*cm/s*bar] | SK HSA [%] | SK YKL-40 [%] |
|---|---|---|---|
| Theranova 400, unsterile (9) | 213 | 1.29 | 32.9 |
| MCO-Ci (4) | 251 | 2, 17 | 44,8 |
| #5-WKB-001-01 (9) | 113 | 0,25 | 13,8 |

As can be seen in comparison with the values provided for SCs in **Table 2 and 4,** coating a base membrane with a process according to the invention results in an improvement of the selectivity of the base membrane, which is especially prominent with regard to the increase in the SC for YKL-40. Such improvement is achieved by e-beam irradiating the membrane in the presence of the coating solution ("filled" state) or in the "wet" state. However, irradiation in the "filled" state is preferable.

**Example 5.3: Influence of irradiation type and dose: gamma versus e-beam radiation**

The influence of the e-beam dose (25 kGy versus 75 kGy) was again tested with minimodules in different states (dry, wet, filled) that are coated with solutions having the same chondroitin concentration (20 mg/mL). **Table 6** shows the results of these tests in terms of the sieving coefficients for HSA and YKL-40 achieved in each case. The results confirm that a higher irradiation dose in combination with a module that is filled with the coating solution during irradiation (bold) surprisingly provides for the best results, even though lower doses (see e.g., #5) are also effective, and also wet modules still show an effect.

**Table 6: Coating of MCO-Ci 400 membranes according to the invention with different e-beam doses (25 kGy or 75 kGy) and in different states. "MM" refers to "Minimodule".**

| # | Membrane in MM (State) | E-Beam Dose (kGy) | No. of MM tested | Lp [E-4*cm/s*bar] | SK HSA [%] | SK YKL-40 [%] |
|---|---|---|---|---|---|---|
| 1 | MCO-Ci 400 (dry) | 25 | 1 | 254 | 1, 66 | 46,2 |
| **2** | **MCO-Ci 400 (filled)** | **75** | **3** | **169** | **1,75** | **64,0** |
| 3 | MCO-Ci 400 (wet) | 75 | 3 | 344 | 3, 83 | 59,6 |
| 4 | MCO-Ci 400 (wet) | 25 | 3 | 274 | 2, 95 | 55,3 |
| 5 | MCO-Ci 400 (filled) | 25 | 3 | 178 | 2,31 | 68,5 |

**Table 7** shows the different impact of the irradiation type. Using e-beam radiation in the coating process is more effective in comparison to gamma irradiation. Coating was performed with a chondroitin solution of 20 mg/mL with Biosynth CS-A.

**Table 7: Minimodules with MCO Ci-400 membranes were irradiated with either gamma or e-beam (bold) under otherwise same conditions. The coating solution contained chondroitin in a concentration of 20 mg/mL. The Lp is shown in [$10^{-4}$ cm/s*bar]. "SC" refers to "Sieving Coefficient", "HSA" refers to "Human Serum Albumin". The values are average values based on three measurements each.**

| # | Membrane | Coating Conditions | Irradiation | Lp | SC HSA [%] | SC YKL-40 [%] |
|---|---|---|---|---|---|---|
| 1 | MCO Ci-400 | 25 kGy, wet | Gamma | 443 | 5.70 | 63.1 |
| 2 | MCO Ci-400 | 25 kGy, filled | Gamma | 416 | 6.37 | 67.9 |
| 3 | **MCO Ci-400** | **25 kGy, wet** | **E-Beam** | **274** | **2.95** | **55.3** |
| 4 | **MCO Ci-400** | **25 kGy, filled** | **E-Beam** | **178** | **2.31** | **68.5** |

Selected membranes of **Table 6 and Table 7** are depicted in **Figure 5** with their SC values for HSA and YKL-40. In **Figure 5,** the position of the data point is a measure of the impact of the coating and selectivity. As can be seen, the best effect on selectivity increase is obtained with filled devices during e-beam treatment (data points A and B corresponding to minimodules #5 and #2 of **Table 6,** respectively). Data points C, D, and E correspond to minimodules #1, #4, and #3, respectively, of **Table 6.** These minimodules were submitted to e-beam treatment in dry and wet states. Data points $\gamma1$ and $\gamma2$ correspond to minimodules #1 and #2 of **Table 7,** respectively, showing that gamma irradiation does not have the same beneficial effect as e-beam treatment. MCO Ci-400 is the base membrane without coating.

**Example 5.4: Chondroitin Type CS-C**

For comparison with chondroitin CS-A (Biosynth, MW 10-30 kDa), membranes were coated with chondroitin of the CS-C type (Chondroitin sulfate C sodium salt from Biosynth) on MCO Ci-400 base membranes at chondroitin concentrations of 40 mg/mL or 80 mg/mL, as indicated, and e-beam treated with a dose of 75 kGy each. **Table 8** shows the effects of the coating also in comparison with water-filled devices without chondroitin. As can be seen from the HSA values, both CS-A and CS-C can be used to modify the sieving coefficients and selectivity of a base membrane. A stronger concentration effect is visible for CS-C, wherein an increase of the chondroitin concentration in the coating solution results in a clearly improved (lower) SC for HSA.

**Table 8: Membranes were coated with chondroitin of the CS-A and CS-C type according to the invention as indicated. Lp [\*$10^{-4}$cm/(bar\*s)] and sieving coefficients [%] have been measured as described above. The first two modules were only filled with water to show the effect of chondroitin. All modules were irradiated in the filled state as indicated. "n" indicates the number of modules tested, "SD" means "standard deviation". The values for Lp and SC are average values ("AV").**

| Membrane (MM) | Coating | E-Beam Irradiation (dose) | Lp | | | SC HSA [%] | | | SC YKL-40 [%] | | |
|---|---|---|---|---|---|---|---|---|---|---|---|
| | | | n | AV | SD | n | AV | SD | n | AV | SD |
| MCO Ci-400 | Water (filled) | 75 kGy | 3 | 339 | 22 | 3 | 5.50 | 0.41 | - | - | - |
| MCO Ci-400 | Water (filled) | 75 kGy | 4 | 287 | 12 | 3 | 5.98 | 0.81 | - | - | - |
| MCO Ci-400 | 40 mg/mL CS-A (filled) | 75 kGy | 4 | 138 | 4 | 3 | 1.01 | 0.34 | - | - | - |
| MCO Ci-400 | 40 mg/mL CS-A (filled) | 75 kGy | 4 | 118 | 4 | 3 | 1.05 | 0.13 | - | - | - |
| MCO Ci-400 | 40 mg/mL CS-C (filled) | 75 kGy | 4 | 153 | 11 | 3 | 1.19 | 0.08 | - | - | - |
| MCO Ci-400 | 80 mg/mL CS-C (filled) | 75 kGy | 4 | 112 | 8 | 3 | 0.45 | 0.05 | - | - | - |

**Example 5.5: Effects on YKL-40 clearance and albumin loss**

To demonstrate the influence of the coating and shift in sieving coefficients on albumin loss and clearance for YKL-40 in filter modules, tests were carried out according to **Examples 1.5 and 1.7.** For the tests, filter devices were used instead of minimodules. **Table 9** shows the results on albumin loss and clearance for YKL-40, which confirm the effects already demonstrated with minimodules for sieving coefficients. The coating with chondroitin (CS-A from Biosynth, MW 10-30 kDa) results in a significant reduction of albumin loss of the MCO CI-400 membrane. At the same time, the clearance of YKL-40 remains very high, resulting in a very efficient filter device for the selective removal of larger middle molecules while being able to excellently retain albumin.

**Table 9: Albumin loss and YKL-40 clearance of filter devices containing membranes of the invention and comparative devices/membranes. "n" indicates the number of modules tested, "SD" means "standard deviation", "AV" refers to "average value".**

| Membrane (Filter) | Coating | E-Beam Irradiation | Albumin Loss [g/60 min] | | | Clearance [ml/min] YKL-40 | |
|---|---|---|---|---|---|---|---|
| | | | n | AV | SD | AV | SD |
| MCO Ci-400 | No chondroitin (filled with $H_2O$) | 75 kGy | 3 | 1.3 | 0.12 | 59.7 | 2.3 |
| **MCO Ci-400** | **40 mg/mL (filled)** | **75 kGy** | **3** | **0,3** | **0,0** | **59,1** | **0,7** |
| **MCO Ci-400** | **40 mg/mL (filled)** | **75 kGy** | **3** | **0,5** | **0,1** | **60,0** | **4,4** |
| Theranova 400 | N/A | N/A | 1 | 0,5 | N/A | 28, 6 | N/A |

The results shown in **Table 9** are visualized in **Figure 6.** It is clearly visible that a coating with chondroitin (e.g., 40 mg/mL chondroitin in the coating solution) followed by e-beam treatment at e.g., 75 kGy in the filled state leads to a significant reduction in albumin loss, whereas the clearance of YKL-40 remains stable.

**Example 6: Stability of chondroitin on the membrane**

Further tests were carried out to determine the stability of chondroitin on the coated membrane and the amount of chondroitin which can be extracted from the coated membrane. Some extraction or elution of chondroitin from the coating is fully acceptable as chondroitin sulfate is a compound which is present naturally in the body as an essential part of hyaline cartilage. Chondroitin is used as a chondroprotective drug for the treatment of osteoarthritis. It is suggested in the literature that chondroitin is partially absorbed by the human body and reaches the joints, thus leading to a reduction in pain and slowing down of the rate of joint destruction and cartilage loss (Zhu et al.: Effectiveness and safety of glucosamine and chondroitin for the treatment of osteoarthritis: a meta-analysis of randomized controlled trials. Journal of Orthopedic Surgery and Research (2018), 13:170). Chondroitin was further found to have an excellent safety profile (Monfort et al.: Chondroitin sulphate for symptomatic osteoarthritis: critical appraisal of meta-analyses. Curr Med Res Opin. (2008); 24(5):1303). The recommended oral dose per day is 800-1200 mg per day. Therefore, it is important to understand how much chondroitin is eluted from a coating according of the invention.

Filter modules with MCO Ci-400 hollow fiber membrane bundles (1.7 $m^2$) were coated (lumen) with a chondroitin solution containing 40 mg/mL chondroitin (CS-A, average MM 10-30 kDa) and an e-beam irradiation dose of 75 kGy. The filter module was emptied after irradiation and rinsed with ultrapure water at 50°C in recirculation mode (QB=300 mL/min) for 10 minutes in an *in vitro* treatment model, i.e., without undergoing final sterilization. After rinsing, elution during dialysis treatment was simulated in the same setup (recirculation mode, QB = 300 ml/min) at 40°C with 500 ml of ultrapure water for 4h. As a reference, a Theranova 400 filter (1.7 $m^2$) was submitted to a rinsing and elution process as described for the test filter. After 4h of simulated treatment the amount of chondroitin that was eluted from the test filter and the Theranova 400 filter was determined with a DMMB assay as mentioned above. **Figure 7** shows the results for four measurements. 6.0 mg chondroitin are eluted with water per device in 4h of simulated treatment (standard deviation 2.6). As to be expected, no chondroitin is detected in the assay when using Theranova 400.

**Claims**

1.  A membrane coated with chondroitin for use in extracorporeal blood treatment applications, wherein the membrane is prepared by treating a base membrane with an aqueous solution comprising from 0.1 wt.-% to 10.0 wt.-% of chondroitin sulfate (CS), followed by submitting the coated membrane to e-beam radiation at a dose of from 12.5 kGy to 115 kGy, and wherein the coated membrane is **characterized by** a higher selectivity in comparison to the base membrane.

2.  The membrane according to claim 1, wherein the base membrane comprises (i) a blend of a polysulfone (PS), polyethersulfone (PES), or polyarylethersulfone (PAES), and polyvinylpyrrolidone (PVP), preferably in an amount of from 0.5 wt.-% to 5.0 wt.-% PVP and from 95.0 wt.-% to 99.5 wt.-% PES, PS, or PAES.

3.  The membrane according to claim 1 or claim 2, wherein the base membrane is treated with an aqueous solution comprising from 0.1 wt.-% to 8.0 wt.-% chondroitin sulfate.

4.  The membrane according to any of claims 1 to 3, wherein the base membrane is coated with chondroitin sulfate in an amount of from 0.30 $\mu g/cm^2$ to 1.20 $\mu g/cm^2$ of the membrane area.

5.  The membrane according to any of claims 1 to 4, wherein the base membrane after treatment with an aqueous solution comprising chondroitin sulfate is e-beam treated with a dose of from 12.5 kGy to 110 kGy.

6.  The membrane according to any of claims 1 to 5, wherein the base membrane has a MWRO of from 9.0 to 14.0 kDa and a MWCO of from 60.0 to 130.0 kDa as measured by dextran sieving before blood contact according to the method defined in the description.

7.  The membrane according to any of claims 1 to 5, wherein the base membrane has a MWRO of from 5.0 kDa to 8.5 kDa and a MWCO of from 25 kDa to 50 kDa as measured by dextran sieving before blood contact according to the method defined in the description.

8. The membrane according to any of claims 1 to 5, wherein the base membrane has a MWRO of from 15kDa to 20kDa and a MWCO of from 170kDa to 320kDa as measured by dextran sieving before blood contact according to the method defined in the description.

9. The membrane according to any of claims 1 to 8 wherein the base membrane is prepared from a polymer solution which comprises from 10 to 15 wt.%, relative to the total weight of the polymer solution, of polysulfone, polyethersulfone or polyarylethersulfone, and from 1 to 10 wt.%, relative to the total weight of the polymer solution, of polyvinylpyrrolidone.

10. The membrane according to any of claims 1 to 9, wherein the e-beam radiation is carried out at a dose of from 20 kGy to 100 kGy.

11. The membrane according to any of claims 1 to 10, wherein the membrane is a hollow fiber membrane or a flat sheet membrane.

12. The membrane according to any of claims 1 to 11, wherein the chondroitin sulfate is chondroitin-4-sulfate (CS-A).

13. The membrane according to any of claims 1 to 12, wherein the chondroitin sulfate and has an average molecular weight (MW) of between 10 and 50 kDa.

14. A process for preparing a membrane according to claim 1, comprising the steps of

(a) Providing a base membrane;
(b) Providing an aqueous chondroitin solution comprising chondroitin in an amount of from between 10 mg/mL and 100 mg/mL;
(c) Contacting the base membrane with the solution of step (b); and
(d) Submitting the membrane of step (c) to e-beam irradiation at a dose of from 12.5 kGy to 110 kGy without drying of the membrane.

15. The process of claim 14, wherein the base membrane comprises a blend of a polysulfone (PS), polyethersulfone (PES), or polyarylethersulfone (PAES), and polyvinylpyrrolidone (PVP).

16. The process of claim 14 or claim 15, wherein the membrane is submitted to the e-beam radiation in the presence of the solution of step (b).

17. A medical device for use in blood treatment applications, wherein the medical device comprises a membrane according to any of claims 1 to 13.

18. The medical device of claim 17, wherein the medical device is a hemodialyzer.

19. A method of increasing the selectivity of a membrane, comprising the steps of

(a) Contacting the membrane with an aqueous solution of chondroitin having a chondroitin concentration of between 10 mg/mL and 100 mg/mL;
(b) Irradiating the membrane of step (a) with e-beam at a dose of from 12.5 to 110 kGy without drying of the membrane.

20. A method according to claim 19, wherein step (b) is carried out in the presence of the solution of step (a).

Figure 1

Hollow Fiber

A

Coating Step

Chondroitin Coating Solution

B

E-beam radiation

C

Figure 2

Figure 3

Figure 4

Figure 5

Figure 6

Figure 7

Europäisches
Patentamt

European
Patent Office

Office européen
des brevets

# EUROPEAN SEARCH REPORT

**Application Number**

EP 23 21 6323

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 4 201 508 A1 (GAMBRO LUNDIA AB [SE]) 28 June 2023 (2023-06-28) * table VII on p. 23; paragraphs [0070], [0104], [0071], [0103] * * the whole document * | 1-20 | INV. B01D67/00 A61M1/16 B01D69/02 B01D69/08 |

-----

TECHNICAL FIELDS
SEARCHED      (IPC)

B01D
A61M

The present search report has been drawn up for all claims

| Place of search | Date of completion of the search | Examiner |
|---|---|---|
| Munich | 8 April 2024 | Hennebrüder, K |

EPO FORM 1503 03.82 (P04C01)

# EP 4 570 364 A1

## ANNEX TO THE EUROPEAN SEARCH REPORT
## ON EUROPEAN PATENT APPLICATION NO.

EP 23 21 6323

This annex lists the patent family members relating to the patent documents cited in the above-mentioned European search report.
The members are as contained in the European Patent Office EDP file on
The European Patent Office is in no way liable for these particulars which are merely given for the purpose of information.

08-04-2024

| Patent document cited in search report | | Publication date | Patent family member(s) | | Publication date |
|---|---|---|---|---|---|
| EP 4201508 | A1 | 28-06-2023 | EP | 4201508 A1 | 28-06-2023 |
| | | | WO | 2023117812 A1 | 29-06-2023 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015118046 A1
- WO 2015118045 A1
- WO 2017030502 A1
- WO 2023117812 A1
- WO 2004056460 A1

**Non-patent literature cited in the description**

- **BOWRY** ; **CHAZOT**. The scientific principles and technological determinants of haemodialysis membranes. *Clin Kidney J,* 2021, vol. 14 (4), i5-i16
- **BOSCHETTI-DE-FIERRO et al.** *Scientific Reports*, 2015, vol. 5, 18448
- **ROSNER et al.** Classification of Uremic Toxins and Their Role in Kidney Failure. *CJASN*, 2021, vol. 16, 1-8
- **LORENZ et al.** *Kidney International*, 2018, vol. 93, 221-230
- **KIRSCH et al.** *Nephrology Dialysis Transplantation*, 2017, vol. 32, 165
- **MIRAGLIA et al.** *Food and Chemical Toxicology*, 2016, vol. 93, 89-101
- **VOLPI**. *molecules*, 2019, vol. 24, 1447
- **AWOFIRANYE et al.** *Fermentation*, 2022, vol. 8, 323
- **NING et al.** *Progress in Polymer Science*, 2018, vol. 81, 144-162
- **REN et al.** *Chemical Engineering Journal*, 2019, vol. 370, 1027-1038
- **SCHULZE et al.** *Macromolecular Rapid Communications*, 2010, vol. 31, 467-472
- **RONCO** ; **CLARK**. *Nature Reviews. Nephrology*, 2018, vol. 14 (6), 394-410
- **GONDOUIN** ; **HUTCHISON**. High Cut-Off Dialysis Membranes: Current Uses and Future Potential. *Advances in Chronic Kidney Disease*, 2011, vol. 18, 180-187
- **ROSNER et al.** Classification of Uremic Toxins and Their Role in Kidney Failure. *Clin J Am Soc Nephrol.*, 2021
- **SAID et al.** A Review of Commercial Developments and Recent Laboratory Research of Dialyzers and Membranes for Hemodialysis Application. *Membranes*, 2021, vol. 11, 767
- **GONDOUIN** ; **HUTCHISON**. High Cut-Off Dialysis Membranes: Current Uses and Future Potential.. *Advances in Chronic Kidney Disease*, 2011, vol. 18, 180-187
- **FARNDALE et al.** *Biochimica et Biophysica Acta*, 1986, vol. 883, 173-177
- **ZHU et al.** Effectiveness and safety of glucosamine and chondroitin for the treatment of osteoarthritis: a meta-analysis of randomized controlled trials.. *Journal of Orthopedic Surgery and Research*, 2018, vol. 13, 170
- **MONFORT et al.** Chondroitin sulphate for symptomatic osteoarthritis: critical appraisal of meta-analyses. *Curr Med Res Opin*, 2008, vol. 24 (5), 1303